# EUROPEAN PATENT SPECIFICATION

(11) **EP 2 209 494 B1**
(45) Date of publication and mention of the grant of the patent: **20.07.2016**
(21) Application number: 08806549.5
(22) Date of filing: 08.10.2008
(51) Int. Cl.: A61K 47/48

(54) **NOVEL CONJUGATED PROTEINS AND PEPTIDES**
NEUARTIGE KONJUGIERTE PROTEINE UND PEPTIDE
NOUVELLES PROTÉINES ET NOUVEAUX PEPTIDES CONJUGUÉS

(30) Priority: 09.10.2007 EP 07253992; 21.07.2008 GB 0813339
(43) Date of publication of application: 28.07.2010
(73) Proprietor: Polytherics Limited, Babraham Cambridge CB22 3AT (GB)
(72) Inventor: BROCCHINI, Stephen, Welling Garden City AL8 6LA (GB); BRYANT, Penny, Kent DA14 4DL (GB); CONG, Yuehua, London N12 9RT (GB); CHOI, Ji-Won, Essex RM9 6TA (GB); GODWIN, Antony, London E18 1PB (GB); POWELL, Keith, London E14 7LE (GB)
(74) Representative: Scott, Susan Margaret
(86) International application number: PCT/GB2008/003409
(87) International publication number: WO 2009/047500

(56) References cited:
- WO-A-95/34326
- WO-A-99/32134
- WO-A-2005/007197
- BALAN SIBU ET AL: "Site-specific PEGylation of protein disulfide bonds using a three-carbon bridge" BIOCONJUGATE CHEMISTRY, vol. 18, no. 1, January 2007 (2007-01), pages 61-76, XP002470617 ISSN: 1043-1802
- MASRI M S ET AL: "Protein reactions with methyl and ethyl vinyl sulfones" JOURNAL OF PROTEIN CHEMISTRY, vol. 7, no. 1, February 1998 (1998-02), pages 49-54, XP008080477 ISSN: 0277-8033
- LIBERATORE F A ET AL: "SITE-DIRECTED CHEMICAL MODIFICATION AND CROSS-LINKING OF A MONOCLONAL ANTIBODY USING EQUILIBRIUM TRANSFER ALKYLATING CROSS-LINK REAGENTS" BIOCONJUGATE CHEMISTRY, ACS, WASHINGTON, DC, US, vol. 1, no. 1, 1990, pages 36-50, XP002313939 ISSN: 1043-1802
- ROBERTS M J ET AL: "Chemistry for peptide and protein PEGylation" ADVANCED DRUG DELIVERY REVIEWS, AMSTERDAM, NL, vol. 54, no. 4, 17 June 2002 (2002-06-17), pages 459-476, XP002293146 ISSN: 0169-409X
- LEE ET AL: "Tumor pH-responsive flower-like micelles of poly(l-lactic acid)-b-poly(ethylene glycol)-b-poly(l-histidine)" JOURNAL OF CONTROLLED RELEASE, ELSEVIER, AMSTERDAM, NL, vol. 123, no. 1, 25 September 2007 (2007-09-25), pages 19-26, XP022265747 ISSN: 0168-3659

## Description

This invention relates to a process for the preparation of novel conjugated proteins and peptides.

Many therapeutically active molecules, for example proteins, do not possess the properties required to achieve efficacy in clinical medical use. For example, many native proteins do not make good medicines because upon administration to patients there are several inherent drawbacks that include: (1) proteins are digested by many endo- and exo-peptidases present in blood or tissue; (2) many proteins are immunogenic to some extent; and (3) proteins can be rapidly excreted by kidney ultrafiltration and by endocytosis. Some molecules which might find utility as active therapeutic agents in medicines are systemically toxic or lack optimal bioavailability and pharmacokinetics. When proteins clear from the blood circulation quickly they typically have to be administered to the patient frequently. Frequent administration further increases the risk of toxicity, especially immunologically derived toxicities.

Water soluble, synthetic polymers, particularly polyalkylene glycols, are widely used to conjugate therapeutically active molecules such as proteins. These therapeutic conjugates have been shown to alter pharmacokinetics favourably by prolonging circulation time and decreasing clearance rates, decreasing systemic toxicity, and in several cases, displaying increased clinical efficacy. The process of covalently conjugating polyethylene glycol, PEG, to proteins is commonly known as "PEGylation".

It is important for optimised efficacy and to ensure dose to dose consistency that the number of conjugated polymer molecules per protein is the same for each molecule, and that each polymer molecule is specifically covalently conjugated to the same amino acid residue in each protein molecule. Nonspecific conjugation at sites along a protein molecule results in a distribution of conjugation products and, frequently, unconjugated protein, to give a complex mixture that is difficult and expensive to purify.

WO 2005/007197 describes a series of novel conjugation reagents. These can be used to react with nucleophilic groups in a biological molecule, for example a protein, to produce a protein-polymer conjugate. These reagents find particular utility for their ability to conjugate with both sulphur atoms derived from a disulfide bond in a protein to give novel thioether conjugates.

Immobilized-metal affinity chromatography (IMAC) is a standard technique for the purification of proteins and peptides. In this technique, a polyhistidine tag, often referred to as a "his-tag" (a short chain of contiguous histidine residues, typically 5 or 6 residues, not present in the native protein) is attached by synthetic methods to a protein, generally to one of the termini of the amino acid chain. The resulting protein or peptide is said to be "histidine tagged". Polyhistidine tags bind strongly to metals such as nickel and cobalt, via at least two of the histidine residues. In IMAC, polyhistidine tagged proteins are passed over a nickel- or cobalt-containing column. The polyhistidine tag binds strongly to the column, thus enabling the tagged protein to be separated from mixtures. Polyhistidine tags are widely used, being attached to a wide range of proteins and peptides to enable them or products derived therefrom to be separated from mixtures at a future date.

Some untagged proteins contain histidine residues in close proximity to each other, and such proteins may also bind to nickel and cobalt IMAC columns, although usually less strongly than polyhistidine tagged proteins.

WO 99/32134 discloses histidine-linked protein polymer conjugates. WO 2005/007197 and Balan et al., Bioconj. Chem. 18(1), 61-76, disclose polymer conjugating reagents useful for conjugation across the disulfide bonds of proteins. Masri et al, J. Protein Chem, 7(1), 49-54 discloses the reaction of alkyl vinyl sulfones with histidine, cysteine and lysine groups. WO 95/34326 discloses polymer conjugation reactions using an active sulfone moiety. Liberatore et al, Bioconj. Chem. 1(1), 36-50 discloses a group of protein cross-linkers. Lee et al J. Cont. Release, 123(1), 19-26 discloses miscelles formed from a block copolymer polylactic acid-PEG-polyhistidine. Roberts et al, Adv. Drug Del Rev. 54(4), 459-476 is a review article concerning PEGylation.

We have now found an improved method of conjugating a polymer, especially PEG, to proteins and peptides. In this method, the polymer is conjugated to histidine residues in a polyhistidine tag. The reagents of WO 2005/007197 act as extremely efficient and stable conjugating agents for proteins and peptides containing polyhistidine tags to produce novel conjugates.

Accordingly, the present invention provides a process of conjugating a polymer to a protein or peptide containing a polyhistidine tag, the process being defined as in claim 1.

The polymer conjugates prepared by the process of the present invention are novel, and form part of the present invention.

Z is a protein or a peptide. Throughout this specification, the term "protein" will be used for convenience, and except where the context requires otherwise, references to "protein" should be understood to be references to "protein or peptide".

A polyhistidine tag is a histidine chain, not found in the native protein or peptide, which has been attached to the protein or peptide by suitable means, for example by chemical means, by post-translational labelling with a polyhistidine tag or a moiety containing a polyhistidine tag, or via protein engineering by inserting histidine sequences in fusion with the target protein, for example by the use of a gene having a short coding sequence which codes for a polyhistidine tag of the desired length.

in which one of X and X' represents a polymer, and the other represents a hydrogen atom;
each Q independently represents a linking group;
W represents an electron-withdrawing moiety or a moiety preparable by reduction of an electron-withdrawing moiety; or, if X' represents a polymer, X-Q-W- together may represent an electron withdrawing group; and in addition, if X represents a polymer, X' and electron withdrawing group W together with the interjacent atoms may form a ring;
Z represents a protein or a peptide linked to A and B via respective histidine residues;
A is a C₁₋₅ alkylene or alkenylene chain; and
B is a bond or a C₁₋₄ alkylene or alkenylene chain.

A further group of novel compounds according to the invention is represented by the general formula:

X-[Q-W-(CH=CH)ᵥ-(CH₂)₂-Z]_{v'} (Ib)

in which X represents a polymer; Q represents a linking group; W represents an electron-withdrawing group or a moiety preparable by reduction of an electron-withdrawing moiety; v represents 0 or an integer of from 1 to 4; v' represent an integer of from 1 to 8; and Z represents a protein or a peptide linked via a polyhistidine tag. In these compounds, v' preferably represents an integer from 1 to 6, preferably 1 to 4, for example 1. Preferably v is 0.

Other reagents which may be used in the process of the present invention include reagents that are capable of acylation or alkylation. Such reagents may be mono or multifunctional and include for example PEG carbonates (e.g. PEG-p-nitrophenyl carbonate, PEG-succinimidyl carbonate, PEG-benzotriazolyl carbonate), PEG carboxylates and PEG esters (e.g. PEG-succinimidyl ester and PEG-p-nitrophenyl ester and their derivatives), PEG aldehyde, PEG-tresyl or -tosyl, PEG-dichlortriazine or -chlorotriazine, PEG vinyl sulfone, PEG maleiimide and PEG-iodoacetamide; and corresponding reagents containing polymers other than PEG.

Z in the novel conjugates of the present invention may be derived from a protein or a peptide. Throughout this specification, the term "protein" will be used for convenience, and except where the context requires otherwise, references to "protein" should be understood to be references to "protein or peptide".

The protein forming part of the novel conjugate of the formula Ia must contain at least two histidine residues. These two residues may be present in the native protein, and if so, the two residues must be located sufficiently close together in the native structure to enable conjugation to the groups A and B. This may occur when two histidine residues are adjacent to each other in the protein chain, or they may be close together as a result of the folding of the protein. Such proteins generally bind to IMAC columns. For example, the prokaryotic organism Escherichia coli has one protein known as YODA where binding to IMAC columns can occur. Preferably, however, the two histidine residues form part of a polyhistidine tag, i.e. a histidine chain, not found in the native protein or peptide, which has been attached to the protein or peptide by suitable means, for example by chemical means, by post-translational labelling with a polyhistidine tag or a moiety containing a polyhistidine tag, or via protein engineering by inserting histidine sequences in fusion with the target protein, for example by the use of a gene having a short coding sequence which codes for a polyhistidine tag of the desired length.

Polyhistidine tags used in the present invention contain up to 12 histidine residues. They must contain at least 2 residues; preferably they contain at least 3 residues, especially from 4 to 10 residues, especially from 5 to 8 residues, for example 5 or 6 residues. They may contain only histidine residues, or they may also contain one or more spacer residues or sequences in addition to histidine residues.

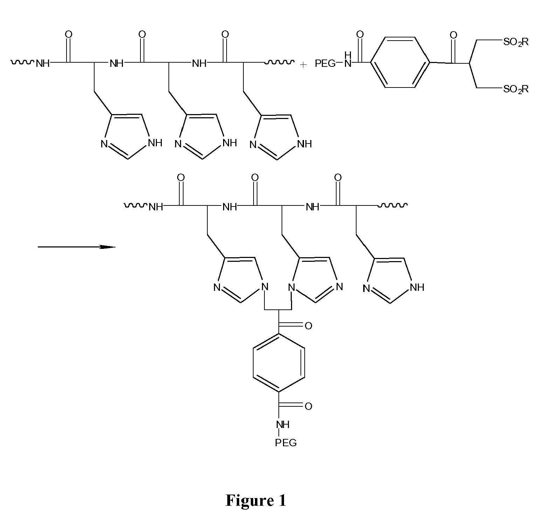

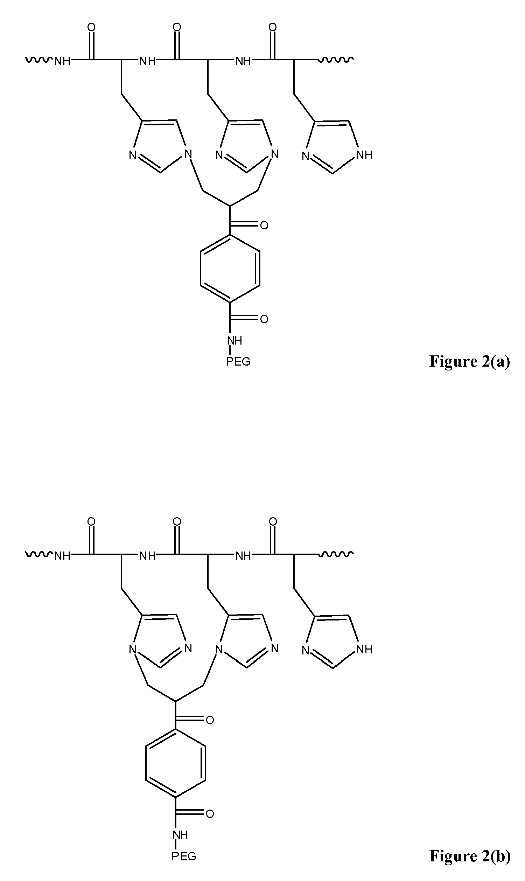

The exact nature of the binding present in the conjugates of the present invention is not currently known. The preparation of one possible conjugate, illustrated using a specific reagent in which each SO₂R represents a leaving group, and in which R preferably represents an alkyl, aryl, alkaryl or aralkyl group, especially a tolyl group, is shown in Figure 1 of the accompanying drawings. Figures 2(a) and 2(b) show alternative conjugates preparable by the process of Figure 1. It is assumed in Figures 1 and 2 that bonding is to adjacent histidine residues, but bonding to spaced apart histidine residues, providing that the spacing is not too large to prevent formation of the conjugate, cannot be ruled out.

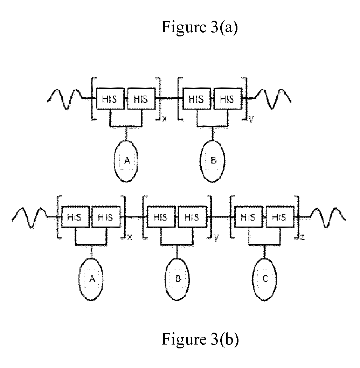

In addition to carrying a polyhistidine tag, the protein may be derivatised or functionalised if desired. In particular, prior to conjugation, the native protein may have been reacted with various blocking groups to protect sensitive groups thereon; or it may have been previously conjugated with one or more polymers or other molecules, either using the process of this invention or using an alternative process. Further, the conjugates of the invention may have one or more additional polymers (including proteins) or other molelcules conjugated to the protein after conjugation according to the invention. Novel conjugates according to the present invention in which the protein is conjugated to one or two additional polymers are shown in Figures 3(a) and3(b) of the accompanying drawings. In these Figures, polymers together with their linking groups are shown schematically as A, B and C, while x, y and z represent the total number of polymers A, B and C conjugated in any combination to the protein via histidine residues. Of course, conjugation of one or more polymers via residues other than histidine is also possible. As well as being conjugated to one or more additional polymers (including proteins), the protein may be conjugated to one or more molecules selected, for example, from small molecules, for example therapeutics or diagnostics; sialic acid; sugars; and starches. B and C in Figure 3 may therefore represent such molecules. In addition, Figure 3 shows A, B and C located adjacent each other, but this is merely schematic, and they may be spaced apart from each other.

Many proteins supplied commercially contain polyhistidine tags, either because the protein has been previously purified by way of IMAC, or to aid future purification of the protein itself or products derived from it. If it is desired to conjugate the protein to a polymer, conjugation to the histidine residues of the polyhistidine tag provides an extremely convenient route, previously not envisaged. Conjugated products, specifically PEGylated products, can be obtained with a high degree of consistency. Use of the conjugating reagents of WO 2005/007197, resulting in conjugation to two histidine residues, results in a macrocycle, leading to a particularly stable and selective conjugate, generally obtained in high yield.

Because the polyhistidine tag is generally attached to the surface of the protein, generally at one end of the protein chain, and can be positioned at any desired site in the protein, the biological activity of the protein is largely maintained following the process for introducing a polyhistidine tag, and also following the subsequent process of site-specific conjugation at histidine residues of the polyhistidine tag to a polymer. For these reasons, the present invention can be used to form conjugates of proteins which have previously proved intractable to traditional conjugation processes.

Provided that a polyhistidine tag contains a sufficient number of histidine residues, a conjugate of the present invention may still be purified using IMAC. For this to be effective, at least two, preferably more, histidine residues in the polyhistidine tag need to remain unconjugated and available for binding to the nickel in the IMAC column. The reduction in the number of free histidine residues post conjugation allows for selectivity in binding to the IMAC column between unconjugated peptide or protein and the conjugated derivative. Where multiple conjugation occurs to the same biological molecule, IMAC allows for separation of the multi-conjugated derivatives.

A polymer in a conjugate of the invention (X or X') may for example be a polyalkylene glycol, a polyvinylpyrrolidone, a polyacrylate, for example polyacryloyl morpholine, a polymethacrylate, a polyoxazoline, a polyvinylalcohol, a polyacrylamide or polymethacrylamide, for example polycarboxymethacrylamide or a polyacrylate or polymethacrylate with phosphatidyl choline pendent groups, such as 2-methacryloyloxyethyl phosphorylcholine, or an HPMA copolymer. Additionally, the polymer may be one which is susceptible to enzymatic or hydrolytic degradation. Such polymers, for example, include polyesters, polyacetals, poly(ortho esters), polycarbonates, poly(imino carbonates), and polyamides, such as poly(amino acids). The polymer may be a homopolymer, random copolymer or a structurally defined copolymer such as a block copolymer. For example it may be a block copolymer derived from two or more alkylene oxides, or from poly(alkylene oxide) and either a polyester, polyacetal, poly(ortho ester), or a poly(amino acid). Polyfunctional polymers that may be used include copolymers of divinylether-maleic anhydride and styrene-maleic anhydride.

Naturally occurring polymers may also be used, for example polysaccharides such as chitin, dextran, dextrin, chitosan, starch, cellulose, glycogen, poly(sialylic acid) and derivatives thereof. A protein may be used as the polymer. This allows conjugation of one protein, for example an antibody or antibody fragment, to a second protein, for example an enzyme or other active protein. Also, if a peptide containing a catalytic sequence is used, for example an O-glycan acceptor site for glycosyltransferase, it allows the incorporation of a substrate or a target for subsequent enzymatic reaction. Polymers such as polyglutamic acid may also be used, as may hybrid polymers derived from natural monomers such as saccharides or amino acids and synthetic monomers such as ethylene oxide or methacrylic acid.

If the polymer is a polyalkylene glycol, this is preferably one containing C₂ and/or C₃ units, and is especially a polyethylene glycol. A polymer, particularly a polyalkylene glycol, may contain a single linear chain, or it may have branched morphology and can be composed of many chains either small or large. The so-called Pluronics are an important class of PEG block copolymers. These are derived from ethylene oxide and propylene oxide blocks. Substituted polyalkylene glycols, for example methoxypolyethylene glycol, may be used. In a preferred embodiment of the invention, a single-chain polyethylene glycol is initiated by a suitable group, for example an alkoxy, e.g. methoxy, aryloxy, carboxy or hydroxyl group, and is connected at the other end of the chain to a linking group Q or Q' as discussed below.

The polymer may optionally be derivatised or functionalised in any desired way. Reactive groups may be linked at the polymer terminus or end group, or along the polymer chain through pendent linkers; in such case, the polymer is for example a polyacrylamide, polymethacrylamide, polyacrylate, polymethacrylate, or a maleic anhydride copolymer. Multimeric conjugates that contain more than one biological molecule, can result in synergistic and additive benefits. If desired, the polymer may be coupled to a solid support using conventional methods.

The optimum molecular weight of the polymer will of course depend upon the intended application. Preferably, the number average molecular weight is in the range of from 250g/mol, for example 500g/mole, to around 75,000g/mole. When the compound of the general formula I is intended to leave the circulation and penetrate tissue, for example for use in the treatment of inflammation caused by malignancy, infection or autoimmune disease, or by trauma, it may be advantageous to use a lower molecular weight polymer in the range 2000-30,000g/mole. For applications where the compound of the general formula I is intended to remain in circulation it may be advantageous to use a higher molecular weight polymer, for example in the range of 20,000 - 75,000g/mole.

The polymer to be used should be selected so the conjugate is soluble in the solvent medium for its intended use. For biological applications, particularly for diagnostic applications and therapeutic applications for clinical therapeutic administration to a mammal, the conjugate will be soluble in aqueous media. Many proteins such as enzymes have utility in industry, for example to catalyze chemical reactions. For conjugates intended for use in such applications, it may be necessary that the conjugate be soluble in either or both aqueous and organic media. The polymer should of course not unduly impair the intended function of the protein.

Preferably the polymer is a synthetic polymer, and preferably it is a water-soluble polymer. The use of a water-soluble polyethylene glycol is particularly preferred for many applications.

A linking group Q' or Q may for example be a direct bond, an alkylene group (preferably a C₁₋₁₀ alkylene group), or an optionally-substituted aryl or heteroaryl group, any of which may be terminated or interrupted by one or more oxygen atoms, sulphur atoms, -NR groups (in which R has the meaning given below), keto groups, -O-CO- groups and/or -CO-O- groups. Suitable aryl groups include phenyl and naphthyl groups, while suitable heteroaryl groups include pyridine, pyrrole, furan, pyran, imidazole, pyrazole, oxazole, pyridazine, primidine and purine. The linkage to the polymer may be by way of a hydrolytically labile bond, or by a non-labile bond.

Substituents which may be present on an optionally substituted aryl or heteroaryl group include for example one or more of the same or different substituents selected from -CN, -NO₂, -CO₂R, -COH, -CH₂OH, -COR, -OR, -OCOR, -OCO₂R, -SR, -SOR, -SO₂R, -NHCOR, -NRCOR, -NHCO₂R, -NR'CO₂R, -NO, -NHOH, -NR'OH, -C=N-NHCOR, -C=N-NR'COR, -N⁺R₃, -N⁺H₃, -N⁺HR₂, -N⁺H₂R, halogen, for example fluorine or chlorine, -C=CR, -C=CR₂ and -C=CHR, in which each R or R' independently represents a hydrogen atom or an alkyl (preferably C₁₋₆ alkyl) or an aryl (preferably phenyl) group. The presence of electron withdrawing substituents is especially preferred.

Preferably the group Q which links to a hydrogen atom in a compound of the formula Ia is an alkylene group or a direct bond. Most preferably, X in formula Ia is a polymer, and X'-Q- is H-.

W may for example represent a keto or aldehyde group CO, an ester group -O-CO- or a sulphone group -SO₂-, or a group obtained by reduction of such a group, e.g. a CH.OH group, an ether group CH.OR, an ester group CH.O.C(O)R, an amine group CH.NH₂, CH.NHR or CH.NR₂, or an amide CH.NHC(O)R or CH.N(C(O)R)₂. If X-Q-W- together represent an electron withdrawing group, this group may for example be a cyano group.

The precise conditions used in the conjugation process of the invention will of course depend upon which conjugation reagent is being used, and which protein or peptide is being conjugated. Such conditions are within the common knowledge of the skilled man. For example, the pH for the reaction will generally be between pH 4 to pH 10, typically between about 6 and about 8.5, for example about 6.5 to 8.0, preferably about 7.0-7.5. The concentration of the protein or peptide in the reaction mixture will generally be above 0.20 mg/ml and typically above 0.4 mg/ml, for example 0.5 mg/ml to 1.5 mg/ml. reagents of WO 2005/007197, the process comprises reacting either (i) a compound of the general formula 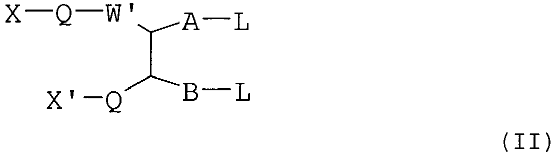
in which one of X and X' represents a polymer and the other represents a hydrogen atom;
Q represents a linking group;
W' represents an electron-withdrawing group, for example a keto group, an ester group -O-CO- or a sulphone group -SO₂-; or, if X' represents a polymer, X-Q-W' together may represent an electron withdrawing group;
A represents a C₁₋₅ alkylene or alkenylene chain;
B represents a bond or a C₁₋₄ alkylene or alkenylene chain; and
each L independently represents a leaving group;
or (ii) a compound of the general formula 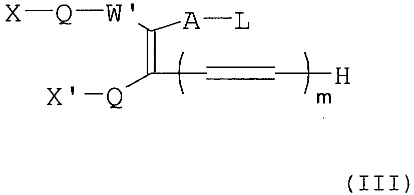 in which X, X', Q, W', A and L have the meanings given for the general formula II, and in addition if X represents a polymer, X' and electron-withdrawing group W' together with the interjacent atoms may form a ring, and m represents an integer 1 to 4; with a protein or a peptide containing at least two histidine residues.

The conjugation reagents II and III are chemically equivalent to each other, and are described in WO 2005/007197. They are characterised by having a cross-functionalised, latently cross-conjugated, bis-alkylating moiety that is selective for two nucleophiles. It is surprising that high selectivity to two histidine residues may be obtained. Other conjugation methods tend to produce mixtures of products; for example, it has previously been found difficult to bind selectively to histidine residues rather than to lysine residues.

The or each leaving group L may for example represent -SR, -SO₂R, -OSO₂R, -N⁺R₃, -N⁺HR₂, -N⁺H₂R, halogen, or -OØ, in which R has the meaning given above, and Ø represents a substituted aryl, especially phenyl, group, containing at least one electron withdrawing substituent, for example -CN, -NO₂, -CO₂R, -COH, -CH₂OH, -COR, -OR, -OCOR, -OCO₂R, -SR, -SOR, -SO₂R, -NHCOR, -NRCOR, -NHCO₂R, -NR'CO₂R, -NO, -NHOH, -NR'OH, -C=N-NHCOR,-C=N-NR'COR, -N⁺R₃, -N⁺HR₂, -N⁺H₂R, halogen, especially chlorine or, especially, fluorine, -C=CR, -C=CR₂ and -C=CHR, in which R and R' have the meanings given above.

Typical structures in which W' and X' together form a ring include 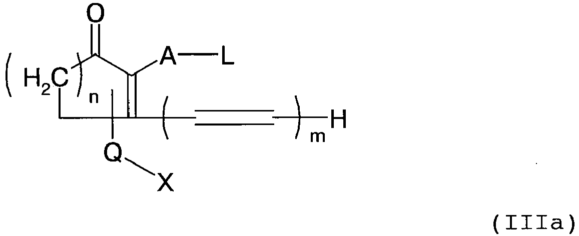 in which n is an integer from 1 to 4, and

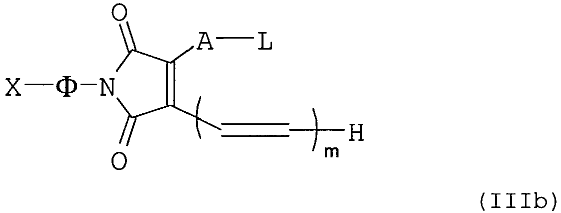

In one preferred embodiment the process according to the invention uses a reagent of the general formula: 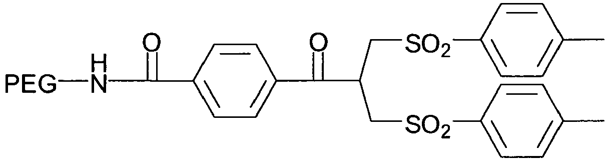 to produce a novel conjugate of the general formula:

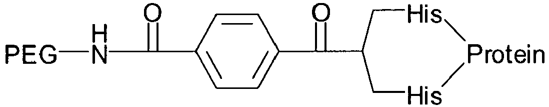

A key feature of this embodiment of the process of this invention is that an α-methylene leaving group and a double bond are cross-conjugated with an electron withdrawing function that serves as a Michael activating moiety. If the leaving group is prone to elimination in the cross-functional reagent rather than to direct displacement and the electron-withdrawing group is a suitable activating moiety for the Michael reaction then sequential intramolecular bis-alkylation can occur by consecutive Michael and retro-Michael reactions. The leaving moiety serves to mask a latent conjugated double bond that is not exposed until after the first alkylation has occurred and bis-alkylation results from sequential and interactive Michael and retro-Michael reactions as described in J. Am. Chem. Soc. 1979, 101, 3098-3110 and J. Am. Chem. Soc. 1988, 110, 5211-5212.). The electron withdrawing group and the leaving group are optimally selected so bis-alkylation can occur by sequential Michael and retro-Michael reactions. It is also possible to prepare cross-functional alkylating agents with additional multiple bonds conjugated to the double bond or between the leaving group and the electron withdrawing group as described in J. Am. Chem. Soc. 1988, 110, 5211-5212.

Some examples of novel conjugates according to the invention include the following:

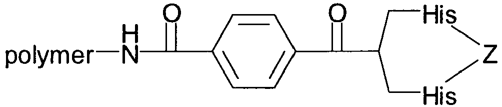

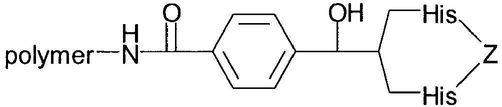

Some examples of reagents which can be used in the process according to the invention include the following:

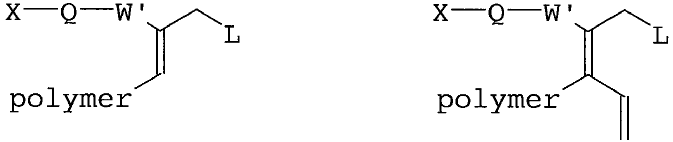

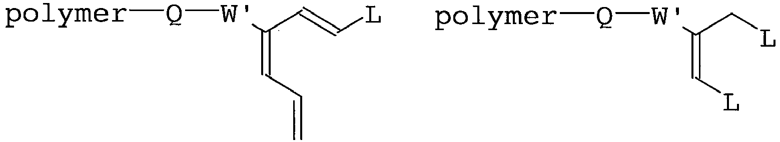

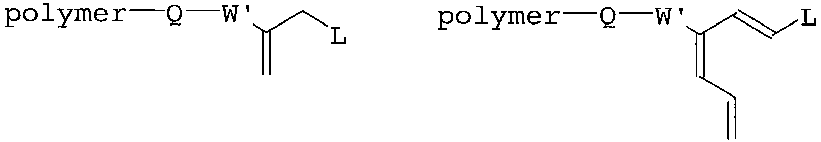

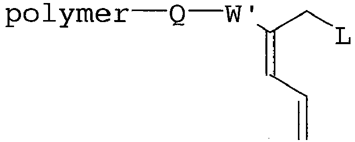

With respect to the protein, the histidine residues are positioned in a polyhistidine tag. Of course, polymers other than polyethylene glycol may replace the PEG in the above formulae. Without wishing to be bound by theory, the bonding to the histidine residues may be as shown in the following formula: 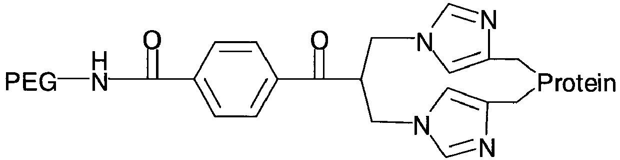 but bonding to other nitrogen atoms in the histidines is also possible.

The immediate product of the above process is a compound of the general formula Ia in which W is an electron-withdrawing group. Such compounds have utility in themselves; because the process of the invention is reversible under suitable conditions, additionally compounds of formula Ia in which W is an electron-withdrawing moiety have utility in applications where release of the free protein is required, for example in direct clinical applications. An electron-withdrawing moiety W may, however, be reduced to give a moiety which prevents release of the protein, and such compounds will also have utility in many clinical, industrial and diagnostic applications. Further, the fact that once W has been reduced reverse reactions can no longer occur, means that no exchange will be observed if a stronger nucleophile is added. Therefore, it becomes possible, for example, to PEGylate via a polyhistidine tag; reduce W; then reduce a disulfide bond in the protein; and carry out a subsequent PEGylation across the reduced disulfide bond. For some proteins, it may be possible to carry out such a process without reducing the group W.

Thus, for example, a moiety W containing a keto group may be reduced to a moiety W containing a CH(OH) group; an ether group CH.OR may be obtained by the reaction of a hydroxy group with an etherifying agent; an ester group CH.O.C(O)R may be obtained by the reaction of a hydroxy group with an acylating agent; an amine group CH.NH₂, CH.NHR or CH.NR₂ may be prepared from a ketone or aldehyde by reductive amination); or an amide CH.NHC(O)R or CH.N(C(O)R)₂ may be formed by acylation of an amine). A group X-Q-W- which is a cyano group may be reduced to an amine group. formed by acylation of an amine). A group X-Q-W- which is a cyano group may be reduced to an amine group.

The process may be carried out in a solvent or solvent mixture in which all reactants are soluble. The protein may be allowed to react directly with the compound of the general formula II or III in an aqueous reaction medium. This reaction medium may also be buffered, depending on the pH requirements of the nucleophile. The optimum pH for the reaction will generally be at least 6.0, typically between about 6.8 and about 8.5, for example about 6.5 or 7.0 to 8.0, for example about 7.5-8.0 but preferably about 7.0 to 7.5. It is surprising that the process according to the invention is selective to binding to histidine residues in a polyhistidine tag at basic pH, as literature methods of PEGylation tend to suggest that basic conditions lead to non-selective PEGylation, including PEGylation to lysine residues, and that PEGylation to histidine is optimised at pH 6.0 to 6.5. Even then, literature methods suggest non-selective PEGylation, including PEGylation to lysine. The optimal reaction conditions will of course depend upon the specific reactants employed, but in general, use of a pH towards the lower end of the above range will tend to increase selectivity towards histidine binding, while use of a pH towards the upper end of the above range will tend to increase the yield.

Reaction temperatures between 3-37°C are generally suitable: proteins may decompose or denature impairing function if the conjugation reaction is conducted at a temperature where these processes may occur. Reactions conducted in organic media (for example THF, ethyl acetate, acetone) are typically conducted at temperatures up to ambient.

The protein can be effectively conjugated with the desired reagent using a stoichiometric equivalent or a slight excess of reagent, unlike many other reagents. However, since the reagents do not undergo competitive reactions with aqueous media used to solvate proteins, it is possible to conduct the conjugation reaction with an excess stoichiometry of reagent. The excess reagent and the product can be easily separated by ion exchange chromatography during routine purification of proteins, or by separation using nickel.

As mentioned above, depending on the number of histidine residues present, more than one polymer may be conjugated to a suitable protein, and this is shown schematically in Figure 3. For example, two polymers could be conjugated to a 6-residue polyhistidine tag. This provides a useful method of attaching multiple polymer residues to a protein. This may be desired when it is wished to obtain a high molecular weight product using a lower molecular weight PEG, e.g. to obtain a 60kD molecular weight product using three 20kD PEG chains; or when it is desired to add two different polymers, for example a protein and a PEG, to another protein to obtain multiple effects; or to achieve, for example, glycosylation and PEGylation in a single step. Such multiple conjugation is often difficult to achieve with conventional conjugation technology.

The compounds of the general formula Ia have a number of applications. They may for example be used for direct clinical application to a patient, and accordingly, the present invention further provides a pharmaceutical composition comprising a compound of the invention together with a pharmaceutically acceptable carrier. The invention further provides a compound of the invention for use in therapy, and a method of treating a patient which comprises administering a pharmaceutically-effective amount of a compound or a pharmaceutical composition according to the invention to the patient. Any desired pharmaceutical effect, for example trauma treatment, enzyme replacement, protein replacement, wound management, toxin removal, antiinflammatory, anti-infective, immunomodulatory, vaccination or anti-cancer, may be obtained by suitable choice of protein.

The compounds of the invention may also be used in non-clinical applications. For example, many physiologically active compounds such as enzymes are able to catalyse reactions in organic solvents, and compounds of the invention may be used in such applications. Further, compounds of the invention may be used as diagnostic tools. Compounds of the invention may include an imaging agent, for example a radio nucleotide, to enable tracking of the compound *in vivo.*

The protein may for example be a peptide, polypeptide, antibody, antibody fragment, enzyme, cytokine, chemokine, receptor, blood factor, peptide hormone, toxin, transcription protein, or multimeric protein.

The following gives some specific proteins which may have utility in the present invention, depending upon the desired application. Enzymes include carbohydrate-specific enzymes, proteolytic enzymes and the like. Enzymes of interest, for both industrial (organic based reactions) and biological applications in general and therapeutic applications in particular include the oxidoreductases, transferases, hydrolases, lyases, isomerases and ligases disclosed by US 4,179,337. Specific enzymes of interest include asparaginase, arginase, adenosine deaminase, superoxide dismutase, catalase, chymotrypsin, lipase, uricase, bilirubin oxidase, glucose oxidase, glucuronidase, galactosidase, glucocerebrosidase, glucuronidase, glutaminase

The proteins used in compounds of the present invention include for example factor VII, VIII or IX and other blood factors, insulin, ACTH, glucagen, somatostatin, somatotropins, thymosin, parathyroid hormone, pigmentary hormones, somatomedins, erythropoietin, luteinizing hormone, hypothalamic releasing factors, antidiuretic hormones, prolactin, interleukins, interferons, colony stimulating factors, hemoglobin, cytokines, antibodies, chorionicgonadotropin, follicle-stimulating hormone, thyroid-stimulating hormone and tissue plasminogen activator.

Certain of the above proteins such as the interleukins, interferons and colony stimulating factors also exist in non-glycosylated form, usually the result of preparation by recombinant protein techniques. The non-glycosylated versions may be used in the present invention.

Other proteins of interest are allergen proteins disclosed by Dreborg et al Crit. Rev. Therap. Drug Carrier Syst. (1990) 6 315 365 as having reduced allergenicity when conjugated with a polymer such as poly(alkylene oxide) and consequently are suitable for use as tolerance inducers. Among the allergens disclosed are Ragweed antigen E, honeybee venom, mite allergen and the like.

Glycopolypeptides such as immunoglobulins, ovalbumin, lipase, glucocerebrosidase, lectins, tissue plasminogen activator and glycosilated interleukins, interferons and colony stimulating factors are of interest, as are immunoglobulins such as IgG, IgE, IgM, IgA, IgD and fragments thereof.

Of particular interest are receptor and ligand binding proteins and antibodies and antibody fragments which are used in clinical medicine for diagnostic and therapeutic purposes. The antibody may used alone or may be covalently conjugated ("loaded") with another atom or molecule such as a radioisotope or a cytotoxic/anti-infective drug. Epitopes may be used for vaccination to produce an immunogenic polymer - protein conjugate.

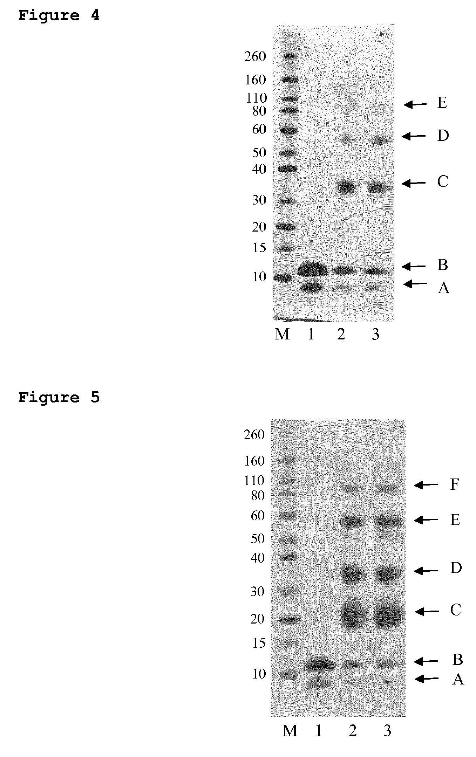

The following Examples illustrate the invention. Results of the Examples are given in the accompanying Figures 4 to 12and 14 to 20. In the Examples, proteins pro-BNP containing a polyhistidine tag of 6 histidine residues, and beta synuclein containing a polyhistidine tag of 8 histidine residues were used. In cardiac tissue, brain natriuretic peptide (BNP) is synthesized as a 134 amino acid precursor (prepro-BNP), which is cleaved by proteases to form a 108 amino acid pro-BNP. Beta synuclein is a small, soluble protein found primarily in brain tissue, and has a chaperone-like activity. Other proteins used are Interferon α-2b, endostatin, an anti-TNF alpha domain antibody fragment.

### Example 1: 12 kDa PEGylation of His₆-proBNP

### PEG reagent structures:

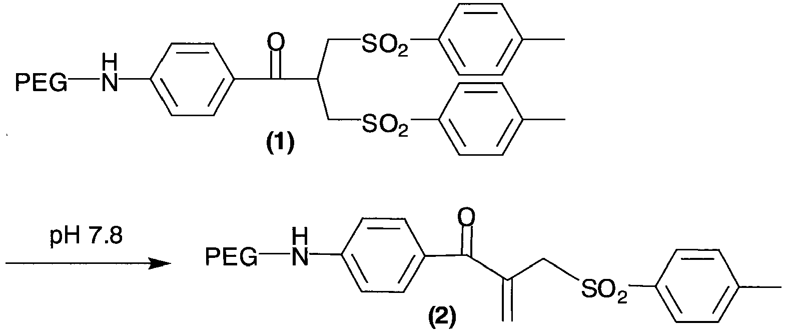

12 kDa PEG mono-sulfone (structure (2)) was prepared by incubation of the PEG bis-sulfone (structure (1)) in pH 7.8, 50 mM sodium phosphate buffer (10 mg/mL) for 5 hours. Two aliquots of C-terminal 6 × histidine tagged ProBNP (Abcam, ab51402, 13 kDa) in pH 7.8, 50 mM sodium phosphate (10 µL, 0.5 mg/mL) were chilled on ice and then either 1 molar equivalent (with respect to protein concentration) or 3 molar equivalents of 12 kDa PEG mono-sulfone were added (1.6 µL or 4.8 µL respectively). The reaction mixtures were then placed in the fridge and left for 16 hours.

The resulting reaction mixtures were analysed using SDS-PAGE for size-dependent separation of their constituents and the resulting gel after staining with Instant blue stain (Novexin) is shown in Figure 4. In Figure 4, the lane labelled M shows the Novex Sharp protein markers (Invitrogen) used as calibrants. The band labelled B in lane 1 is the sample of His₆-proBNP as supplied (Abcam, 1 mg/mL). Band A is an impurity. Lane 2 is obtained from the 1 equivalent of 12 kDa PEG reaction mixture and shows bands, labelled C, D and E which correspond to a mono-PEGylated product, di-PEGylated product and tri-PEGylated product respectively. The lower bands, labelled A and B correspond to unreacted His₆-proBNP and the impurity in the supplied sample respectively. Lane 3 is obtained from the 3 equivalents of 12 kDa PEG reaction mixture and shows bands, labelled C, D and E which correspond to a mono-PEGylated product, di-PEGylated product and tri-PEGylated product respectively. The lower bands, labelled B and A correspond to unreacted His₆-proBNP and the impurity in the supplied sample respectively.

The gel was then also stained with barium iodide for visualisation of PEG with the result shown in Figure 5. The bands in Lane 1 correspond to unreacted PEG reagent which is labelled C, and bands labelled D, E and F which correspond to a mono-PEGylated product, di-PEGylated product and tri-PEGylated product respectively. The lower bands, labelled B and A correspond to unreacted His₆-proBNP and the impurity in the supplied sample respectively. Lane 3 which is obtained from the 3 equivalents of 12 kDa PEG reaction mixture shows a band labelled C which corresponds to unreacted PEG reagent and bands labelled D, E and F which correspond to a mono-PEGylated product, di-PEGylated product and tri-PEGylated product respectively. The lower bands, labelled B and A correspond to unreacted His₆-proBNP and the impurity in the supplied sample respectively.

### Example 2: 30 kDa PEGylation of His₆-proBNP

30 kDa PEG *mono*-sulfone (structure **(2)** from example 1) was prepared by incubation of the PEG *bis*-sulfone (structure **(1)** from example 1) in pH 7.8, 50 mM sodium phosphate buffer (10 mg/mL) for 5 hours. Two aliquots of C-terminal 6 × histidine tagged ProBNP (Abcam, ab51402) in pH 7.8, 50 mM sodium phosphate (10 µL, 0.5 mg/mL) were chilled on ice then either 1 molar equivalent (with respect to protein concentration) or 3 equivalents of 30 kDa PEG mono-sulfone were added (2.0 µL or 6.0 µL respectively). The reaction was then placed in the fridge and left for 16 hours.

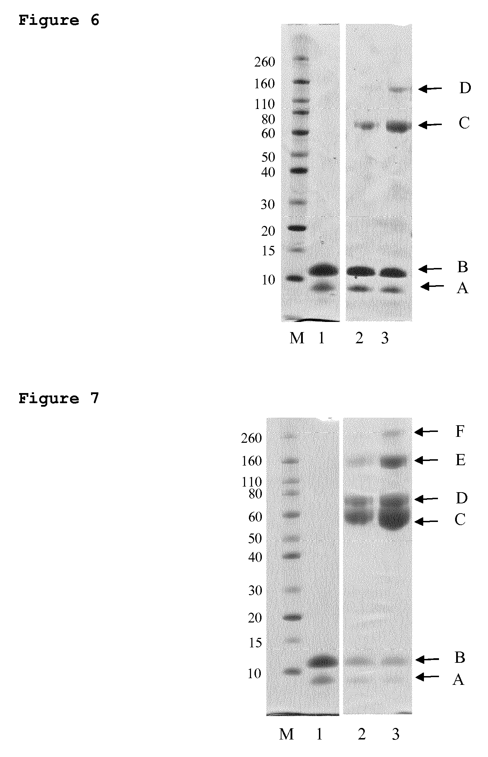

The resulting reaction mixtures were analysed using SDS-PAGE for size-dependent separation of its constituents and the resulting gel after staining with Instant blue (Novexin) is shown in Figure 6. The lane labelled M shows the Novex Sharp protein markers (Invitrogen) used as calibrants. The band labelled B in lane 1 is the sample of His₆-proBNP as supplied (Abcam, 1 mg/mL). Band A is an impurity. The lane labelled 2 is obtained from the 1 equivalent of 30 kDa PEG reaction mixture and shows a bands labelled C which corresponds to a mono-PEGylated product and lower bands, labelled A and B which correspond to unreacted His₆-proBNP and the impurity in the supplied sample respectively. The lane labelled 3 is obtained from the 3 equivalent of 30 kDa PEG reaction mixture and shows bands, labelled C, and D which correspond to a mono-PEGylated product and di-PEGylated product respectively and lower bands, labelled B and A which correspond to unreacted His₆-proBNP and the impurity in the supplied sample respectively.

The gel was then also stained with barium iodide for visualisation of PEG and the resulting gel is shown in Figure 7. The lane labelled M shows the Novex Sharp protein markers (Invitrogen) used as calibrants. The band labelled B in lane 1 is the sample of His₆-proBNP as supplied (Abcam, 1 mg/mL). Band A is an impurity. The lane labelled 2 is obtained from the 1 equivalent of 30 kDa PEG reaction mixture and shows a band labelled C which corresponds to unreacted PEG reagent, a band labelled F which corresponds to a PEG impurity, bands labelled D and E and F which correspond to mono-PEGylated and di-PEGylated products respectively, and lower bands, labelled B and A which correspond to unreacted His₆-proBNP and the impurity in the supplied sample respectively. The lane labelled 3 is obtained from the 3 equivalents of 30 kDa PEG reaction mixture and shows a band labelled C which corresponds to unreacted PEG reagent, a band labelled F which corresponds to a PEG impurity, bands labelled D and E which correspond to mono-PEGylated and di-PEGylated products respectively, and lower bands, labelled B and A which correspond to unreacted His₆-proBNP and the impurity in the supplied sample respectively.

### Example 3: 12 kDa PEGylation of His₈-beta synuclein and comparative reaction with non-histidine tagged beta synuclein

12 kDa PEG *mono*-sulfone (structure **(2)** from example 1) was prepared by incubation of the PEG *bis*-sulfone (structure **(1)** from example 1) in pH 7.8, 50 mM sodium phosphate buffer (10 mg/mL) for 5 hours. Two aliquots of N-terminal 8 × histidine tagged beta synuclein (Abcam cat. no. ab40545, 15 kDa) in pH 7.8, 50 mM sodium phosphate (10 µL, 0.39 mg/mL) were chilled on ice then either 1 molar equivalent (with respect to protein concentration) or 3 equivalents of 12 kDa PEG mono-sulfone were added (1.6 µL or 4.7 µL respectively). The reaction was then placed in the fridge and left for 16 hours.

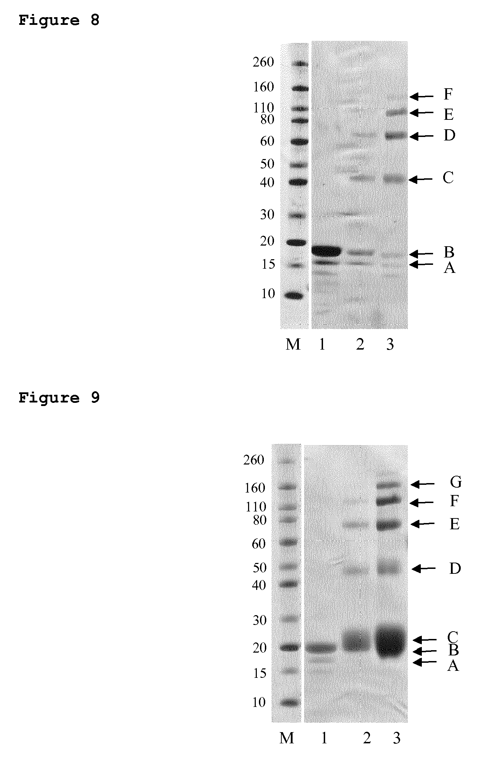

The resulting reaction mixtures were analysed using SDS-PAGE for size-dependent separation of its constituents and the resulting gel after staining with Instant blue (Novexin) is shown in Figure 8. The lane labelled M shows the Novex Sharp protein markers (Invitrogen) used as calibrants.

The band labelled B in lane 1 is the sample of His₈-beta synuclein supplied by Abcam (0.8 mg/mL). Band A is an impurity. The lane labelled 2 is obtained from the 1 equivalent of 12 kDa PEG reaction mixture and shows bands, labelled C and D which correspond to a mono-PEGylated product and di-PEGylated product respectively and lower bands, labelled A and B which correspond to unreacted His₈-beta synuclein and the impurity in the supplied sample respectively. The lane labelled 3 is obtained from the 3 equivalents of 12 kDa PEG reaction mixture and shows bands, labelled C, D, E and F which correspond to a mono-PEGylated product, di-PEGylated product, tri-PEGylated product and tetra-PEGylated product respectively and lower bands, labelled B and A which correspond to unreacted His₈-beta synuclein and the impurity in the supplied sample respectively.

The gel was then also stained with barium iodide for visualisation of PEG and the resulting gel is shown in Figure 9. In Figure 9, the lane labelled M shows the Novex Sharp protein markers (Invitrogen) used as calibrant. The lane labelled 1 is obtained from His₈-beta synuclein as supplied by Abcam. The lane labelled 2 is obtained from the 1 equivalent of 12 kDa PEG reaction mixture and shows bands labelled C, D and E which correspond to a mono-PEGylated product, di-PEGylated product and tri-PEGylated product respectively, and lower bands, labelled B and A which correspond to unreacted His₈-beta synuclein and the impurity in the supplied sample respectively. The lane labelled 3 is obtained from the 3 equivalents of 12 kDa PEG reaction mixture and shows bands labelled D, E and F which correspond to a mono-PEGylated product, di-PEGylated product, tri-PEGylated product and tetra-PEGylated product respectively, and lower bands, labelled B and A which correspond to unreacted His₈-beta synuclein and the impurity in the supplied sample respectively. Band C is a PEG impurity.

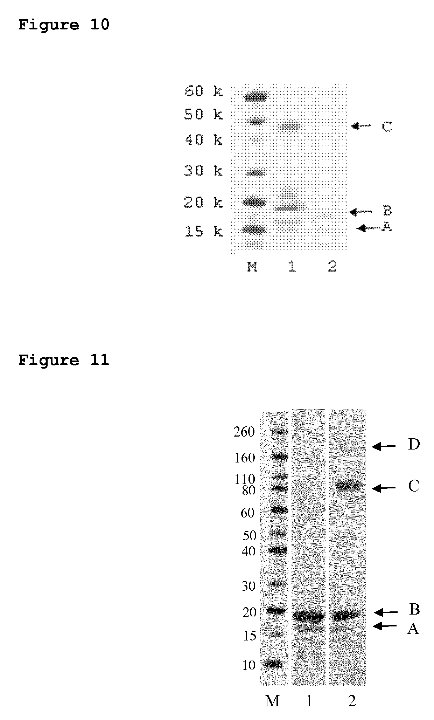

A comparison 12 kDa PEGylation study was performed between His₈-beta synuclein and non-histidine tagged beta synuclein (Abcam. cat no. ab48853) using 1 equivalent of the PEG reagent **(2)** at pH 7.0 and the SDS-PAGE result is shown in Figure 10. The lane labelled M shows the Novex Sharp protein markers (Invitrogen) used as calibrants. The His₈-beta synuclein result is shown in the lane labelled 1. In lane 1, the band labelled B is unreacted His₈-beta synuclein and the band labelled C is mono-PEGylated His₈-beta synuclein. The lane labelled 2 shows the non-histidine tagged beta synuclein reaction and the only visible band is labelled A and is the non-histidine tagged beta synuclein. There is no PEGylated protein band in lane 2.

### Example 4: 30 kDa PEGylation of His₈-beta synuclein

30 kDa PEG *mono*-sulfone (structure **(2)** from example 1) was prepared by incubation of the PEG *bis*-sulfone (structure **(1)** from example 1) in pH 7.8, 50 mM sodium phosphate buffer (10 mg/mL) for 5 hours. An aliquot of N-terminal 8 × histidine tagged beta synuclein (Abcam, ab40545, 15 kDa) in pH 7.8, 50 mM sodium phosphate (10 µL, 0.39 mg/mL) was chilled on ice then 1 molar equivalent (with respect to protein concentration) of 30 kDa PEG *mono*-sulfone was added (1.6 µL). The reaction was then placed in the fridge and left for 16 hours.

The resulting reaction mixture was analysed using SDS-PAGE for size-dependent separation of its constituents and the resulting gel after staining with Instant blue (Novexin) is shown in Figure 11. In Figure 11, the lane labelled M shows the Novex Sharp protein markers (Invitrogen) used as calibrants. The lane labelled 1 is obtained from the sample of His₈-beta synuclein supplied by Abcam (0.8 mg/mL) and the band labelled B is the Polyhistidine tagged protein whilst the band labelled A is an impurity. The lane labelled 2 is obtained from the 1 equivalent of 30 kDa PEG reaction mixture and shows a band labelled C which corresponds to a mono-PEGylated product and lower bands, labelled A and B which correspond to unreacted His₈-beta synuclein and the impurity in the supplied sample respectively.

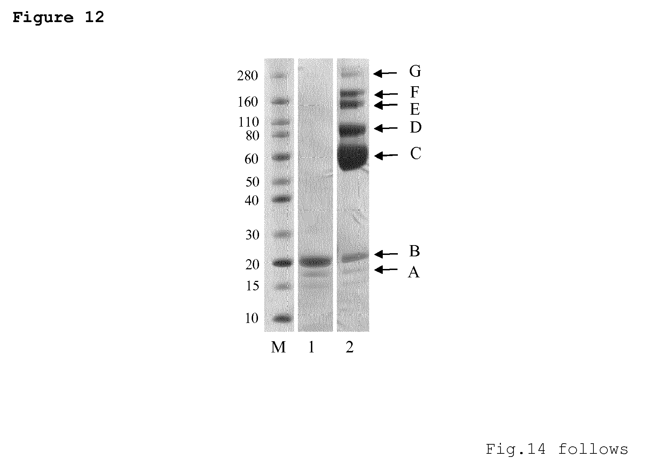

The gel was then also stained with barium iodide for visualisation of PEG and the resulting gel is shown in Figure 12. In Figure 12, the lane labelled M shows the Novex Sharp protein markers (Invitrogen) used as calibrants. The lane labelled 1 is obtained from His₈-beta synuclein as supplied by Abcam. The lane labelled 2 is obtained from the 1 equivalent of 30 kDa PEG reaction mixture and shows a band labelled C which corresponds to unreacted PEG reagent, bands labelled E and G which corresponds to PEG impurities, bands labelled D and F which correspond to a mono-PEGylated product and di-PEGylated product respectively, and lower bands, labelled B and A which correspond to unreacted His₈-beta synuclein and the impurity in the supplied sample respectively.

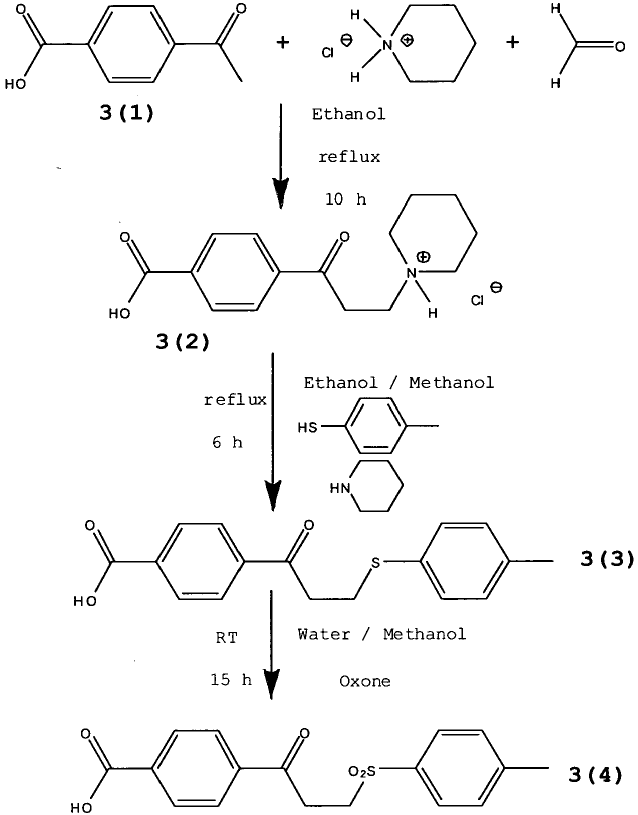

A 250 ml round bottom flask was charged with p-acetylbenzoic acid (15.0 g, **3(1)**), 11.11g piperidine hydrochloride and 8.23g paraformaldehyde. Absolute ethanol (90 ml) and concentrated hydrochloric acid (1 ml) were then added, and the resulting suspension heated under reflux for 10 h while stirring under argon. After stopping the reflux, acetone (150ml) was added and the reaction mixture allowed to cool to room temperature. The resulting white precipitate was isolated on a glass filter (G3) and washed twice with chilled acetone. The solid was then dried under vacuum to give a white crystal powder **(3(2),** 9.72 g). ¹H NMR (400 MHz, DMSO-d₆) δ 1.79, 2.96, 3.45 (br m, CH₂ of piperidine moiety), 3.36 (t, 2H, COCH₂), 3.74 (t, 2H, NCH₂), 8.09 (m, 4H, Ar*H*).

### Step 2: Synthesis of 4-(3-(p-tolylthio)propanoyl)benzoic acid 3(5).

The *p*-carboxy-3-piperidinopropriophenone hydrochloride **3(2)** (1.0 g) and 4-methylbenzenethiol (417 mg, **3(3))** were suspended in a mixture of absolute ethanol (7.5 ml) and methanol (5 ml). Piperidine (50 µl) was then added and the suspension heated to reflux with stirring for 6 h in an argon atmosphere. The white precipitate afforded after cooling to room temperature was filtered off with a glass filter (G3), washed carefully with cold acetone and dried in vacuum to give **3(3)** (614 mg). ¹H NMR (400 MHz, DMSO-d₆) δ 2.27 (s, 3H, phenyl-C*H*₃), 3.24, 3.39 (t, 2 x 2H, CH₂), 7.14, 7.26 (d, 2 x 2H, Ar*H* of tolyl moiety), 8.03 (m, 4H, Ar*H* of carboxylic acid moiety).

### Step 3, Synthesis of 4-(3-tosylpropanoyl)benzoic acid 3(4).

4-(3-(*p*-tolylthio)propanoyl)benzoic acid **3(4)** (160 mg) was suspended in a mixture of water (10ml) and methanol (10 ml). After cooling in an ice bath, oxone (720 mg, Aldrich) was added and the reaction mixture allowed to warm to room temperature while stirring overnight (15 h). The resulting suspension was diluted with further water (40 ml) so that it became nearly homogeneous and the mixture was then extracted three times with chloroform (in total 100 ml). The pooled chloroform extracts were washed with brine and then dried with MgSO₄. Evaporation of volatiles under vacuum at 30°C afforded a white solid **3(4)** (149 mg). ¹H NMR (400 MHz, DMSO-d₆) δ 2.41 (s, 3H, phenyl-C*H*₃), 3.42 (t, 2H, CO-C*H*₂), 3.64 (t, 2H, SO₂-C*H*₂), 7.46, 7.82 (d, 2 x 2H, Ar*H* of tolyl moiety), 8.03 (m, 4H, Ar*H* of carboxylic acid moiety).

### Step 4, Synthesis of PEGylated 4-(3-tosylpropanoyl)benzoic acid, PEG reagent 3.

The 4-(3-tosylpropanoyl)benzoic acid **3(4)** (133 mg) and 0-(2-aminoethyl)-O'-methyl-PEG (MW 10 kDa, 502 mg, BioVectra) were dissolved in dry toluene (5 ml). The solvent was removed under vacuum without heating and the dry solid residue was then redissolved in dry dichloromethane (15 ml) under argon. To the resulting solution, cooled in an ice bath, was slowly added diisopropylcarbodiimide (DIPC, 60 mg) under argon. The reaction mixture was then kept stirring at room temperature overnight (15 h). Volatiles were then removed under vacuum (30°C, water bath) to afford a solid residue that was redissolved with gentle heating (35°C) in acetone (20 ml). The solution was filtered over non-absorbent cotton wool to remove insoluble material. The solution was then cooled in a dry ice bath to give a white precipitate that was separated by centrifugation (4600 rpm, 30 min). The liquid phase was decanted off and this precipitation procedure was repeated three times. Afterwards the resulting off-white solid was dried under vacuum to afford the PEG reagent **3** (437 mg). ¹H NMR (400 MHz, CDCl₃) δ 2.46 (s,3H, phenyl-C*H*₃), 3.38 (s, 3H, PEG-OC*H*₃), 3.44-3.82 (br m, PEG), 7.38, 7.83 (d, 2 x 2H, Ar*H* of tolyl moiety), 7.95 (m, 4H, Ar*H* of carboxylic acid moiety).

### Step 5: PEGylation on histidine of C-terminal his₈-tagged IFN α-2b

To a 20 µl solution of IFN α-2b (1.13 mg/ml in 10 mM sodium phosphate buffer containing 2 mM EDTA and 150 mM NaCl, pH 7.5) was added 1 molar equivalent of 10 kDa PEG reagent **3** (1.8 µl of a 6 mg/ml solution in deionised water) and the resulting solution incubated overnight at room temperature. A repeat was also performed with 1 molar equivalent of 20 kDa

### Example 6: 5 kDa PEGylation of Hisg-β-synuclein

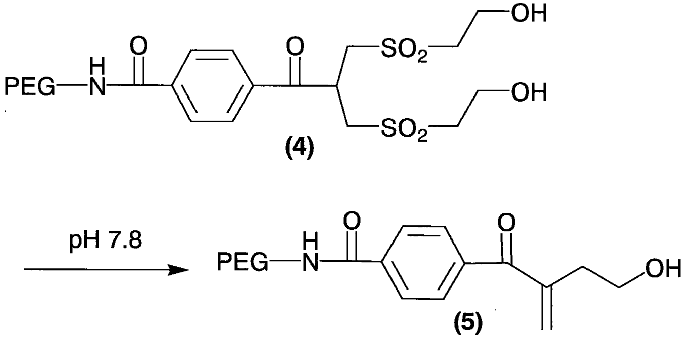

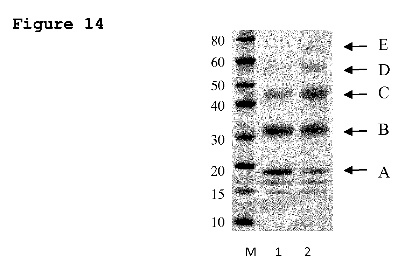

5 kDa PEG *mono*-sulfone (structure **(5))** was prepared by incubation of the PEG *bis*-sulfone (structure **(4))** in pH 7.8, 50 mM sodium phosphate buffer (5 mg/mL) for 3 hours. Two aliquots of N-terminal 8 × histidine tagged beta synuclein (Abcam cat. no. ab40545, 15.4 kDa) in pH 7.4, 50 mM sodium phosphate (10 µL, 0.38 mg/mL) were chilled on ice then either 1 molar equivalent (with respect to protein concentration) or 3 equivalents of 5 kDa PEG mono-sulfone **(5)** were added (0.25 µL or <0.74 µL respectively). As control, two aliquots of beta synuclein (Abcam cat. no. ab48853, 14.3 kDa) were treated the same way and given either 1 molar equivalent (0.18 µL) or 3 equivalents (0.52 µL) of the 5 kDa PEG mono-sulfone. The reactions were then incubated at ambient temperature for 6 h. The resulting reaction solutions were analysed using SDS-PAGE and stained with Instant blue (Expedeon) as shown in Figure **14****.** The lane labelled M shows the Novex Sharp protein markers (Invitrogen) used as calibrants. The lanes labelled 1 and 2 are obtained from the 1 equivalent of 5 kDa PEG reaction mixture and 3 equivalents of PEG reaction mixture respectively. The band labelled A is His₈-beta synuclein. Faint bands below it are impurities from the protein supplied by Abcam. The bands, labelled B, C, D and E which correspond to mono-PEGylated, di-PEGylated, tri-PEGylated and tetra-PEGylated protein products respectively.

### Example 7: 10 kDa PEGylation of His₆-anti TNF-α domain antibody fragment at different pH's

10 kDa PEG *mono*-sulfone (structure **(2)** from example 1) was prepared by incubation of the PEG *bis*-sulfone (structure **(1)** from example 1, 10 mg/mL) in pH 7.8, 50 mM sodium phosphate buffer (5 mg/mL) for 3 h. The C-terminal 6 × histidine tagged anti-TNF alpha domain antibody fragment (12.7 kDa) solution (0.6 mg/mL)in 50 mM sodium phosphate, 150 mM sodium chloride and 2 mM EDTA was prepared at 4 different pH's (pH 6.2, pH 6.7, pH 7.0 and pH 7.4). Three molar equivalents of 10 kDa PEG *mono*-sulfone (**(2),** 1.41 µL) were added to the His₆-domain solutions at each of the four different pH's (10 µL, 0.6 mg/mL). The reactions were then incubated at ambient temperature for 3 hours.

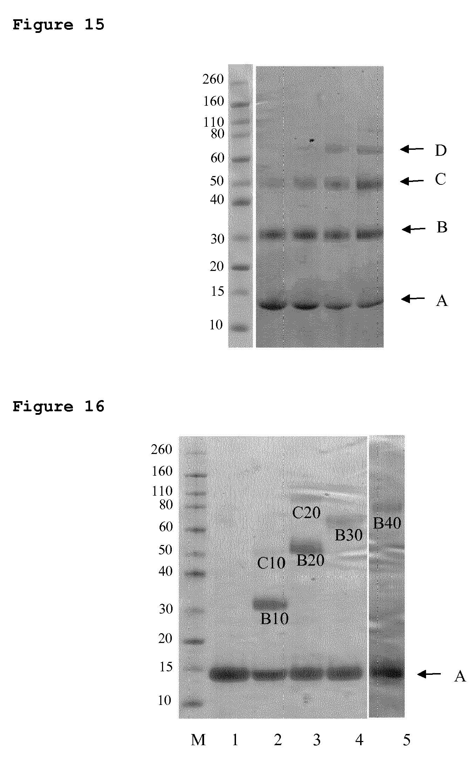

The resulting reaction solutions were analysed using SDS-PAGE and stained with Instant blue (Expedeon) as shown in Figure 15. The lane labelled M shows the Novex Sharp protein markers (Invitrogen). The lanes labelled 1 to 4 are obtained from the reaction mixture using 3 molar equivalents of 10 kDa PEG at pH 6.2, pH 6.7, pH 7.0 and pH 7.4 respectively. The band labelled A is unreacted His₆-anti-TNF alpha domain antibody fragment. The band labelled B is the mono-PEGylated domain product. The bands labelled C and D correspond to di-PEGylated and tri-PEGylated domain fragment respectively. PEGylation is seen at each of the four pH's and the extent of PEGylation increases as the pH is increased.

### Example 8: PEGylation of His₆-anti TNF-α domain antibody fragment using 10 kDa, 20 kDa, 30 kDa and 40 kDa PEG.

10 kDa, 20 kDa, 30 kDa and 40 kDa PEG *bis*-sulfone (structure **(1)** from example 1) were separately incubated in pH 7.8, 50 mM sodium phosphate buffer for 3 hours at ambient temperature to give the corresponding PEG mono-sulfones (structure **(2)** from example 1). The concentration of PEG was 10 mg/mL, 20 mg/mL, 30 mg/mL and 40 mg/mL for 10 kDa, 20 kDa, 30 kDa and 40 kDa PEG respectively. Four aliquots of the C-terminal 6 × histidine tagged anti-TNF domain antibody (12.7kDa)solution (1.25 mg/mL, 5 µL) in 50 mM sodium phosphate, 150 mM sodium chloride and 2 mM EDTA were then added with the 10 kDa, 20 kDa, 30 kDa and 40 kDa PEG mono-sulfone solutions (0.74 µL, 1.5 molar equivalents). The reactions were then incubated at 4°C for 8 hours. The reaction solutions were then analysed by SDS-PAGE as shown in Figure 16. The Lane labelled M shows the Novex sharp protein markers (Invitrogen) used as calibrants. The lane labelled 1 is the His₆- anti TNF domain antibody fragment shown as a reference. The lanes labelled 2 to 5 are obtained from the reaction of 10 kDa, 20 kDa, 30 kDa and 40 kDa PEGs respectively. The bands labelled A are unreacted His₆-anti-TNF domain. The bands labelled B (B10, B20, B30 and B40) correspond to the mono-PEGylated domain fragments for 10, 20, 30 and 40 kDa PEG respectively. The band labelled C10 corresponds to the di-PEGylated product for the 10 kDa PEG reaction and the C20 band corresponds to the di-PEGylated product for the 20 kDa PEG reaction.

### Example 9: 2 kDa PEGylation of His₆-Endostatin and comparative reaction with Endostatin not possessing a polyhistidine tag.

2 kDa PEG *mono*-sulfone (structure **(2)** from example 1) was prepared by incubation of the PEG *bis*-sulfone (structure **(1))** from example 1) in pH 7.8, 50 mM sodium phosphate buffer (1 mg/mL) for 4 hours. Two aliquots of C-terminal 6 × histidine tagged Endostatin (Calbiochem cat. no. 324743) in pH 6.2, 50 mM sodium phosphate (30 µL, 0.5 mg/mL) were chilled on ice then either 1 molar equivalent (with respect to protein concentration) or 3 equivalents of 2 kDa PEG *mono*-sulfone were added (1.4 µL or 4.2 µL respectively). The reaction mixture was then placed in the fridge and left for 16 hours.
Two aliquots of non-tagged Endostatin (Calbiochem cat. no. 324769) in pH 6.2, 50 mM sodium phosphate (10 µL, 0.2 mg/mL) were also chilled on ice, then either 1 molar equivalent (with respect to protein concentration) or 3 equivalents of 2 kDa PEG *mono*-sulfone (0.1 mg/mL) were added (2.0 µL or 6.0 µL respectively). The reaction was then placed in the fridge and left for 16 hours.

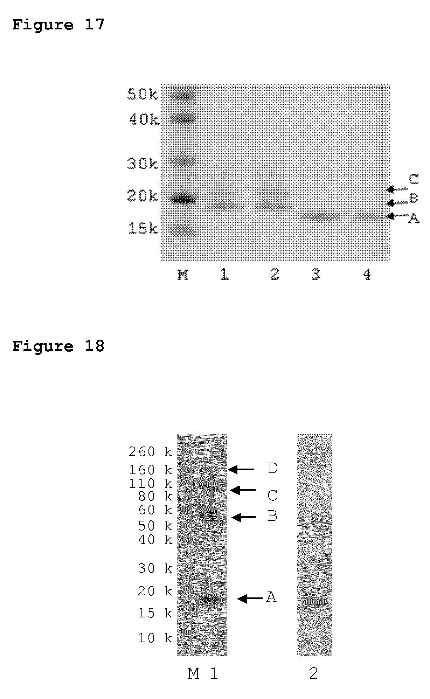

The resulting reaction solutions were analysed using SDS-PAGE for size-dependent separation of their constituents and the resulting gel after staining with Instant blue (Expedeon) is shown in Figure 17. In Figure 17, the left-hand column of spots labelled M shows the Novex Sharp protein markers (Invitrogen). The lane labelled 1 is obtained from the sample of the 1 equivalent of 2kDa PEG with **His₆-Endostatin** reaction and shows bands, labelled B and C which correspond to the unreacted protein, and mono-PEGylated product respectively. The lane labelled 2 is obtained from the sample of the 3 equivalent of 2kDa PEG with His₆-Endostatin reaction and shows bands, labelled B and C which correspond to the unreacted protein and mono-PEGylated product respectively. The lanes labelled 3 and 4 were obtained from the 1 equivalent of 2kDa PEG with Endostatin reaction and 3 equivalents of 2kDa PEG with Endostatin reaction respectively. Only a single band labelled A and B respectively which corresponds to the unreacted protein is present for both reactions showing that the 2 kDa PEG only reacts with the His₆-Endostatin.

### Example 10. 20 kDa and 30 kDa PEGylation of Hi_{S8}-interferon alpha and comparison reaction with non-histidine tagged interferon alpha.

A solution of N-terminal 8 × histidine tagged interferon α-2b (2.63 mL, 1.14 mg/mL) was prepared in pH 7.4, 50 mM sodium phosphate buffer containing 150 mM sodium chloride (PBS) and then 1.7 molar equivalent (with respect to the protein concentration) of 20 kDa PEG bis-sulfone (structure **(1)** from example 1) was added (420 µL of 11.5 mg/mL 20 kDa PEG bis-sulfone solution in deionised water). The reaction was placed in the refrigerator for 18 hours. The resulting reaction mixture was analysed by SDS-PAGE and the result shown in Figure 18. In Figure 18, the lane labelled M shows the Novex Sharp protein markers (Invitrogen) used as calibrants. The lane labelled 1 shows the reaction solution where the band labelled A corresponds to unreacted IFN, the band labelled B to monoPEGylated IFN, the band labelled C to diPEGylated IFN and the band labelled D to triPEGylated IFN.

A second reaction with one equivalent of 30 kDa PEG at 0.5 mg/ml and a solution of N-terminal 8 × histidine tagged interferon α-2b (3.2 mL, 0.793 mg/mL) at pH 7.5. The result was similar to the 20 kDa reaction with a mono-PEGylated IFN band visible between the 60 and 80 kDa protein markers, a band between 110 and 160 kDa corresponding to di-PEGylated IFN and a third band between the 160 and 260 kDa protein markers corresponding to tri-PEGylated IFN. A comparison with non-histidine tagged IFN alpha-2b showed no reaction with one equivalent of PEG reagent **(1)** after 18 h and under the same conditions, with the result shown in lane 2 of Figure 18.

### Example 11. Use of poly(vinyl pyrrolidone) (PVP bis-sulfone) for conjugation to N-terminal 8 × histidine tagged interferon alpha

Preparation of PVP with a terminal amine group: A pressure tube was charged with cysteamine (0.042 g), dioxane (8 ml) and a magnetic stir bar. After gentle heating to allow a solution to form, the solution was purged with argon at room temperature for 5 min. While still purging, 1-vinyl-2-pyrrolidone (2.0 g) was then added and after a further 5 min this was followed by 2,2'-azobis(2-methylpropionitrile) (0.089 g). After a further 2 min the pressure tube was sealed with a screw cap under argon and placed in an oil bath at 60°C for 23 h with stirring. After allowing the tube and contents to cool to room temperature, diethyl ether (15 ml) was added causing precipitation of the polymeric product. The liquid phase was decanted away and the solid residue redissolved in acetone (3 ml). The resulting acetone solution was then added dropwise to rapidly stirring diethyl ether (25 ml) and the precipitate isolated on a no. 2 sintered glass funnel with a slight burst of vacuum. The solid was washed with fresh diethyl ether (10 ml) and then allowed to dry under vacuum at room temperature (mass = 1.24 g, white solid).

Conjugation of protein reactive end group to PVP-amine: PVP-amine (500 mg), 4-[2,2-bis[(p-tolylsulfonyl)methyl]acetyl]-benzoic acid (125 mg), and 4-dimethylaminopyridine (6 mg) were mixed with anhydrous dichloromethane (10 ml) under argon and with stirring was then added 1,3-diisopropylcarbodiimide (80 µl). The resulting mixture was allowed to stir for 20 h at room temperature and then filtered though non-absorbent cotton-wool. To the filtrate was then added diethyl ether (20 ml) and the resulting precipitate isolated by centrifugation (2,000 rpm, 2 min, 2 °C). The liquid phase was decanted off and the remaining residue vortexed with ethyl acetate (5 ml) for several minutes. After decanting off the ethyl acetate phase the solid residue was redissolved in dichloromethane (5 ml) followed by the addition of ethyl acetate (15 ml). The solution was placed in dry-ice for 15 min resulting in precipitation of a solid which was then isolated by centrifugation (2,000 rpm, 2 min, 2 °C). The sticky residue obtained was vortexed with fresh ethyl acetate (5 ml) and then dried under vacuum at room temperature (mass = 0.118 g).

Fractionation of PVP bis-sulfone: The solid material obtained from the above step was mixed with aqueous 20 mM sodium acetate buffer, 150 mM NaCl, pH 4.0 and then centrifuged at 13,000 rpm until a clear solution was visible. The solution phase removed from the solid residue and then fractionated on a HiLoad 16/60 Superdex^{™} 200 prep grade size exclusion column (GE Healthcare) running 20 mM sodium acetate buffer, 150 mM, pH 4.0 at 1 ml/min by collecting fractions every 1 min during peak elution. The fractions eluting between 72 to 80 min was used for protein conjugation.

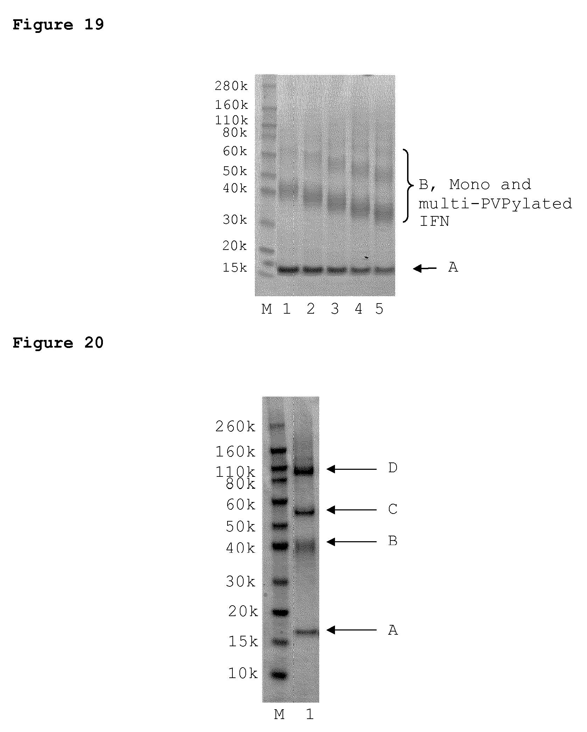

PVP conjugation to N-terminal 8 × histidine tagged IFN alpha: IFN (0.025 mg, 0.5 mg/ml) was prepared in 7.5, 50 mM sodium phosphate buffer containing 150 mM sodium chloride (PBS). To 50 µl of IFN (25 µg, 0.5 mg/ml) was added 50 µl of the fractionated PVP bis-sulfone solution (Fractions 72 min, 74 min, 76 min, 78 min and 80 min). The resulting solution was gently mixed and left at 4°C overnight. The resultant reaction solution was analysed by SDS-PAGE and the result is shown in Figure 19. In Figure 19, the lane labelled M shows the Novex Sharp protein markers (Invitrogen) used as calibrants. The lanes labelled 2 to 5 show the reaction solutions from the different sized PVP reagent fractions used (72 min to 80 min PVP fractions from 2 to 5 respectively). The bands labelled B show the mono- and multi-PVPylated IFN obtained.

### Example 12. Multimeric conjugation of interferon alpha with interferon alpha using N-terminal 8 × histidine tagged Interferon alpha

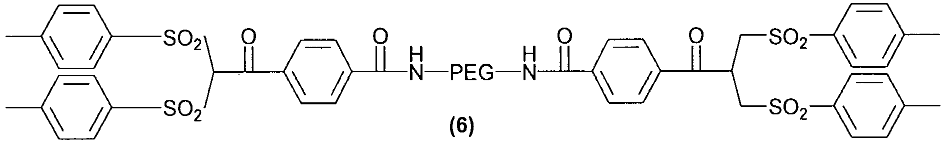

A solution of N-terminal 8 × histidine tagged Interferon α-2b (50 µL, 1.14 mg/mL) was prepared in pH 7.5, 50 mM sodium phosphate buffer containing 150 mM sodium chloride (PBS) and then 0.125 molar equivalents (with respect to the protein concentration) of 10 kDa PEG bifunctional PEG reagent **(6)** was added (0.47 µL of an 8 mg/mL solution of **(6)** in water). The reaction was left at ambient temperature for 18 hours. The resulting reaction mixture was analysed by SDS-PAGE. The resultant gel showed a band between the 50 kDa and 60 kDa Novex sharp protein markers (Invitrogen) after staining with Instant Blue (Expedeon) that corresponds to the IFN-PEG-IFN fusion conjugate.

### Example 13. Multimeric conjugation of N-terminal 8 × histidine tagged Interferon alpha with human serum albumin(HSA) containing a free cysteine.

To a solution of N-terminal 8 × histidine tagged Interferon α-2b (1 mL, 1.14 mg/mL) prepared in pH 7.5, 50 mM sodium phosphate buffer containing 150 mM sodium chloride (PBS) was added human serum albumin (3 molar equivalents to IFN, 10.77 mg) and allowed to dissolve. To this solution was added 10 kDa PEG bis (bis-sulfone) (structure **(6)** from example 12) (1 molar equivalent to IFN, 75 µL of 8 mg/mL 10 kDa PEG bis (bis-sulfone) **(6)** solution in water). The reaction mixture was left at ambient temperature for 18 hours. The resulting reaction mixture was purified by ion-exchange chromatography followed by metal-ion affinity chromatography and then analysed by SDS-PAGE and the result is shown in Figure 20. In Figure 20, the lane labelled M shows the Novex Sharp protein markers (Invitrogen) used as calibrants. The lane labelled 1 shows the purified reaction solution where the band labelled A is unreacted IFN, the band labelled B is mono-PEGylated IFN, the band labelled C is IFN-PEG-IFN and the band labelled D is IFN-PEG-albumin.

## Claims

1. A process for the preparation of a compound of the general formula 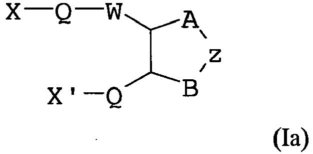
in which one of X and X' represents a polymer, and the other represents a hydrogen atom;
each Q independently represents a linking group;
W represents an electron-withdrawing moiety or a moiety preparable by reduction of an electron-withdrawing moiety; or, if X' represents a polymer, X-Q-W- together may represent an electron withdrawing group; and in addition, if X represents a polymer, X' and electron withdrawing group W together with the interjacent atoms may form a ring;
Z represents a protein or a peptide linked to A and B via histidine residues contained in a polyhistidine tag attached to said protein or peptide and containing from 2 to 12 histidine residues;
A is a C₁₋₅ alkylene or alkenylene chain; and
B is a bond or a C₁₋₄ alkylene or alkenylene chain;
which comprises reacting either (i) a compound of the general formula 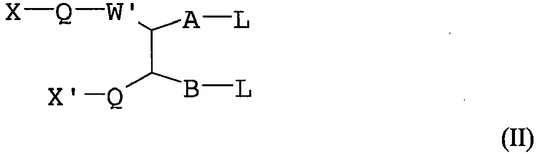 in which:
X, X', Q, A and B have the meanings given above;
W' represents an electron-withdrawing group; or, if X' represents a polymer, X-Q-W' together may represent an electron withdrawing group; and
each L independently represents a leaving group;
or (ii) a compound of the general formula 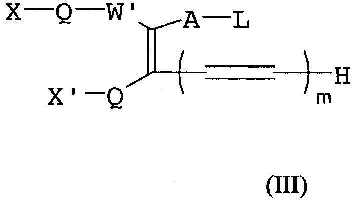 in which X, X', Q, W', A and L have the meanings given for the general formula II, and in addition if X represents a polymer, X' and electron-withdrawing group W' together with the interjacent atoms may form a ring, and m represents an integer 1 to 4; with a protein or a peptide carrying a polyhistidine tag attached to said protein or peptide and containing from 2 to 12 histidine residues; and if desired, reducing the electron-withdrawing group W'.

2. A process as claimed in claim 1, which is carried out at a pH in the range of from 6.8 to 8.5.

3. A process as claimed in either claim 1 or claim 2, in which the polyhistidine tag contains at least 3 histidine residues.

4. A process as claimed in claim 3, in which the polyhistidine tag contains from 4 to 10 histidine residues.

5. A process as claimed in any one of claims 2 to 4, in which the polymer is a polyalkylene glycol, a polyvinylpyrrolidone, a polyacrylate, a polymethacrylate, a polyoxazoline, a polyvinylalcohol, a polyacrylamide, a polymethacrylamide, a HPMA copolymer, a polyester, polyacetal, poly(ortho ester), polycarbonate, poly(imino carbonate), polyamide, a copolymer of an alkylene oxide and a polyester, polyacetal, poly(ortho ester), or poly(amino acid), a polysaccharide, a protein, polyglutamic acid, a copolymer of a saccharide or an amino acid and a synthetic monomer, or a copolymer of divinylether-maleic anhydride and styrene-maleic anhydride.

6. A process as claimed in claim 5, in which the polymer is a polyalkylene glycol.

7. A process as claimed in claim 6, in which the polymer is a polyalkylene glycol containing C₂ and/or C₃ units.

8. A process as claimed in claim 7, in which the polymer is a polyethylene glycol.

9. A process as claimed in claim 5, in which the polymer is a protein.

10. A process as claimed in any one of claims 2 to 9, in which said conjugated protein or peptide is a peptide, polypeptide, antibody, antibody fragment, enzyme, cytokine, chemokine, receptor, blood factor, peptide hormone, toxin, transcription protein, or multimeric protein.

11. A process as claimed in claim 10, in which said conjugated protein or peptide is an antibody or an antibody fragment.

12. A process as claimed in any one or claims 2 to 11, in which each Q independently represents a direct bond, a C₁₋₁₀ alkylene group, or an optionally-substituted aryl or heteroaryl group, any of which may be terminated or interrupted by one or more oxygen atoms, sulphur atoms, -NR groups in which R represents a hydrogen atom or an alkyl or an aryl group, keto groups, -O-CO- groups and/or -CO-O- groups.

13. A process as claimed in claim 12, in which Q is an alkylene group or a direct bond.

14. A process as claimed in any one of claims 2 to 13, in which W represents a keto or aldehyde group CO, an ester group -O-CO- or a sulphone group -SO₂-, or a group obtained by reduction of such a group; or in which X-Q-W- together represent an electron withdrawing group.

15. A process as claimed in any one of claims 2 to 14, in which X is a polymer, and X'-Q- is H-.

16. A compound of the general formula 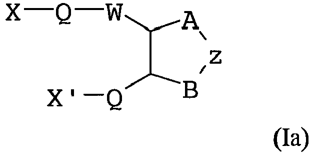
in which one of X and X' represents a polymer, and the other represents a hydrogen atom;
each Q independently represents a linking group;
W represents an electron-withdrawing moiety or a moiety
preparable by reduction of an electron-withdrawing moiety; or, if X' represents a polymer, X-Q-W- together may represent an electron withdrawing group; and in addition, if X represents a polymer, X' and electron withdrawing group W together with the interjacent atoms may form a ring;
Z represents a protein or a peptide linked to A and B via histidine residues contained in a polyhistidine tag attached to said protein or peptide and containing from 2 to 12 histidine residues;
A is a C₁₋₅ alkylene or alkenylene chain; and
B is a bond or a C₁₋₄ alkylene or alkenylene chain;
said compound having been prepared by a process as claimed in any one of claims 1 to 15.

17. A pharmaceutical composition comprising a compound as claimed in claim 16, together with a pharmaceutically acceptable carrier.

18. A compound as claimed in claim 16, or a pharmaceutical composition as claimed in claim 17, for use in therapy.

## Patentansprüche

1. Verfahren zur Herstellung einer Verbindung der allgemeinen Formel: 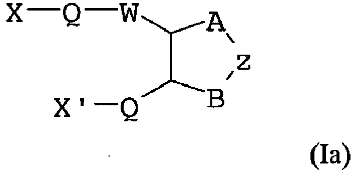
wobei einer von X und X' ein Polymer darstellt, und der andere ein Wasserstoffatom darstellt;
jedes Q unabhängig voneinander eine verbindende Gruppe darstellt;
W einen elektronenziehenden Rest darstellt, oder einen Rest, der durch Reduktion von einem elektronenziehenden Rest herstellbar ist; oder, wenn X' ein Polymer darstellt, X-Q-W-zusammen eine elektronenziehende Gruppe darstellen können; und ausserdem, wenn X ein Polymer darstellt, X' und die elektronenziehende Gruppe W zusammen mit den dazwischenliegenden Atomen einen Ring bilden können;
Z ein Protein oder ein Peptid darstellt, das an A und B über Histidinreste gebunden ist, die in einem Polyhistidin-Tag enthalten sind, das an das Protein oder Peptid gebunden ist und von 2 bis 12 Histidinreste enthält;
A eine C₁₋₅ Alkylen- oder Alkenylen-Kette ist; und
B eine Bindung oder eine C₁₋₄ Alkylen- oder Alkenylen-Kette ist;
welches die Umsetzung umfasst von entweder (i) einer Verbindung der allgemeinen Formel 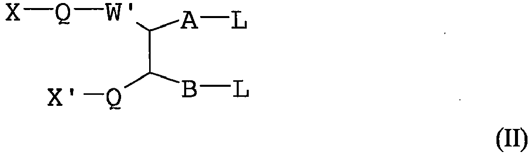 in der
X, X', Q, A und B die oben angegebenen Bedeutungen haben;
W' eine elektronenziehende Gruppe darstellt; oder, wenn X' ein Polymer darstellt, X-Q-W' zusammen eine elektronenziehende Gruppe darstellen können; und
jedes L unabhängig voneinander eine Abgangsgruppe darstellt;
oder (ii) einer Verbindung der allgemeinen Formel 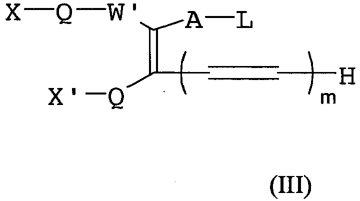 in der X, X', Q, W', A und L die Bedeutungen haben, die für die allgemeine Formel II angegeben sind, und ausserdem, wenn X ein Polymer darstellt, X' und die elektronenziehende Gruppe W' zusammen mit den dazwischenliegenden Atomen einen Ring bilden können, und m eine ganze Zahl von 1 bis 4 darstellt; mit einem Protein oder einem Peptid, das ein Polyhistidin-Tag trägt, das an das Protein oder Peptid gebunden ist und von 2 bis 12 Histidinreste enthält; und, falls gewünscht, Reduzierung der elektronenziehenden Gruppe W'.

2. Verfahren, wie in Anspruch 1 beansprucht, das bei einem pH im Bereich von 6,8 bis 8,5 ausgeführt wird.

3. Verfahren, wie in entweder Anspruch 1 oder Anspruch 2 beansprucht, wobei das Polyhistidin-Tag mindestens 3 Histidinreste enthält.

4. Verfahren, wie in Anspruch 3 beansprucht, wobei das Polyhistidin-Tag von 4 bis 10 Histidinreste enthält.

5. Verfahren, wie in einem der Ansprüche 2 bis 4 beansprucht, wobei das Polymer ein Polyalkylenglykol, ein Polyvinylpyrrolidon, ein Polyacrylat, ein Polymethacrylat, ein Polyoxazolin, ein Polyvinylalkohol, ein Polyacrylamid, ein Polymethacrylamid, ein HPMA Copolymer, ein Polyester, Polyacetal, Polyorthoester, Polycarbonat, Polyiminocarbonat, Polyamid, ein Copolymer aus einem Alkylenoxid und einem Polyester, Polyacetal, Polyorthoester oder einer Polyaminosäure, ein Polysaccharid, ein Protein, Polyglutaminsäure, ein Copolymer aus einem Saccharid oder einer Aminosäure und einem synthetischen Monomer, oder ein Copolymer aus Divinylether-Maleinsäureanhydrid und Styrol-Maleinsäureanhydrid, ist.

6. Verfahren, wie in Anspruch 5 beansprucht, wobei das Polymer ein Polyalkylenglykol ist.

7. Verfahren, wie in Anspruch 6 beansprucht, wobei das Polymer ein Polyalkylenglykol ist, das C₂ und/oder C₃ Einheiten enthält.

8. Verfahren, wie in Anspruch 7 beansprucht, wobei das Polymer ein Polyethylenglykol ist.

9. Verfahren, wie in Anspruch 5 beansprucht, wobei das Polymer ein Protein ist.

10. Verfahren, wie in einem der Ansprüche 2 bis 9 beansprucht, wobei das konjugierte Protein oder Peptid ein Peptid, Polypeptid, Antikörper, Antikörperfragment, Enzym, Cytokin, Chemokin, Rezeptor, Blutfaktor, Peptidhormon, Toxin, Transkriptionsprotein oder multimeres Protein ist.

11. Verfahren, wie in Anspruch 10 beansprucht, wobei das konjugierte Protein oder Peptid ein Antikörper oder ein Antikörperfragment ist.

12. Verfahren, wie in einem der Ansprüche 2 bis 11 beansprucht, wobei jedes Q unabhängig voneinander eine direkte Bindung, eine C₁₋₁₀ Alkylengruppe, oder eine optional substituierte Aryl- oder Heteroarylgruppe darstellt, wobei jede von diesen durch ein oder mehrere Sauerstoffatome, Schwefelatome, Gruppen -NR, in denen R ein Wasserstoffatom oder eine Alkyl- oder eine Arylgruppe darstellt, Ketogruppen, Gruppen -O-CO- und/oder Gruppen -CO-O- terminiert oder unterbrochen sein kann.

13. Verfahren, wie in Anspruch 12 beansprucht, wobei Q eine Alkylengruppe oder eine direkte Bindung ist.

14. Verfahren, wie in einem der Ansprüche 2 bis 13 beansprucht, wobei W eine Keto- oder Aldehydgruppe CO, eine Estergruppe -O-CO- oder eine Sulfongruppe -SO₂- darstellt, oder eine Gruppe, die durch Reduktion einer solchen Gruppe erhalten wird; oder wobei X-Q-W- zusammen eine elektronenziehende Gruppe darstellen.

15. Verfahren, wie in einem der Ansprüche 2 bis 14 beansprucht, wobei X ein Polymer ist und X'-Q- gleich H- ist.

16. Verbindung der allgemeinen Formel 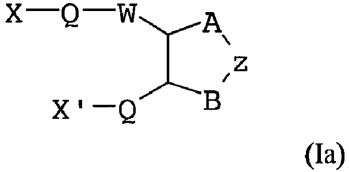
wobei einer von X und X' ein Polymer darstellt, und der andere ein Wasserstoffatom darstellt;
jedes Q unabhängig voneinander eine verbindende Gruppe darstellt;
W einen elektronenziehenden Rest darstellt, oder einen Rest, der durch Reduktion von einem elektronenziehenden Rest herstellbar ist; oder, wenn X' ein Polymer darstellt, X-Q-W-zusammen eine elektronenziehende Gruppe darstellen können; und ausserdem, wenn X ein Polymer darstellt, X' und die elektronenziehende Gruppe W zusammen mit den dazwischenliegenden Atomen einen Ring bilden können;
Z ein Protein oder ein Peptid darstellt, das an A und B über Histidinreste gebunden ist, die in einem Polyhistidin-Tag enthalten sind, das an das Protein oder Peptid gebunden ist und von 2 bis 12 Histidinreste enthält;
A eine C₁₋₅ Alkylen- oder Alkenylen-Kette ist; und
B eine Bindung oder eine C₁₋₄ Alkylen- oder Alkenylen-Kette ist;
wobei die genannte Verbindung durch ein Verfahren hergestellt wurde, wie es in einem der Ansprüche 1 bis 15 beansprucht ist.

17. Pharmazeutische Zusammensetzung, umfassend eine Verbindung, wie in Anspruch 16 beansprucht, zusammen mit einem pharmazeutisch akzeptablen Träger.

18. Eine Verbindung, wie in Anspruch 16 beansprucht, oder eine Pharmazeutische Zusammensetzung, wie in Anspruch 17 beansprucht, für den therapeutischen Einsatz.

## Revendications

1. Procédé pour la préparation d'un composé de formule générale 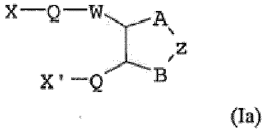
dans laquelle un de X et X' représente un polymère et l'autre représente un atome d'hydrogène ;
chaque Q représente indépendamment un groupe de liaison ;
W représente un groupement électrophile ou un groupement pouvant être préparé par réduction d'un groupement électrophile ; ou bien, si X' représente un polymère, X-Q-W-, conjointement, peuvent représenter un groupe électrophile ; et, en outre, si X représente un polymère, X' et le groupe électrophile W, conjointement avec les atomes intermédiaires, peuvent former un noyau ;
Z représente une protéine ou un peptide lié à A et B par l'intermédiaire de résidus histidine présents dans un marqueur polyhistidine fixé à ladite protéine ou audit peptide et contenant 2 à 12 résidus histidine ;
A représente une chaîne alkylène ou alcénylène en C₁ à C₅ ; et
B représente une liaison ou une chaîne alkylène ou alcénylène en C₁ à c₄ ;
qui comprend la réaction soit (i) d'un composé de formule générale 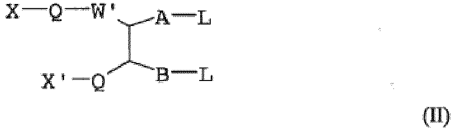 dans laquelle
X, X', Q, A et B répondent aux définitions précitées ;
W' représente un groupe électrophile ; ou, si X' représente un polymère, X-Q-W', conjointement, peuvent représenter un groupe électrophile ; et
chaque L représente indépendamment un groupe partant ;
soit (ii) d'un composé de formule générale 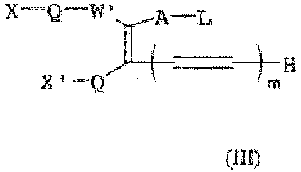 dans laquelle X, X', Q, W', A et L répondent aux définitions indiquées pour la formule générale II et, en outre, si X représente un polymère, X' et le groupe électrophile W', conjointement avec les atomes intermédiaires, peuvent former un noyau, et m représente un nombre entier de 1 à 4 ; avec une protéine ou un peptide portant un marqueur polyhistidine fixé à ladite protéine ou audit peptide et contenant 2 à 12 résidus histidine ; et, si cela est désiré, la réduction du groupe électrophile W'.

2. Procédé suivant la revendication 1, qui est mis en oeuvre à un pH dans la plage de 6,8 à 8,5.

3. Procédé suivant la revendication 1 ou la revendication 2, dans lequel le marqueur polyhistidine contient au moins trois résidus histidine.

4. Procédé suivant la revendication 3, dans lequel le marqueur polyhistidine contient 4 à 10 résidus histidine.

5. Procédé suivant l'une quelconque des revendications 2 à 4, dans lequel le polymère est un polyalkylèneglycol, une polyvinylpyrrolidone, un polyacrylate, un polyméthacrylate, une polyoxazoline, un poly-(alcool vinylique), un polyacrylamide, un polyméthacrylamide, un copolymère HPMA, un polyester, un polyacétal, un poly-(orthoester), un polycarbonate, un poly-(iminocarbonate), un polyamide, un copolymère d'un oxyde d'alkylène et d'un polyester, d'un polyacétal, d'un poly-(orthoester) ou d'un poly-(aminoacide), un polysaccharide, une protéine, un poly-(acide glutamique), un copolymère d'un saccharide ou d'un amino-acide et d'un monomère synthétique ou un copolymère d'éther divinylique-anhydride maléique et de styrène-anhydride maléique.

6. Procédé suivant la revendication 5, dans lequel le polymère est un polyalkylèneglycol.

7. Procédé suivant la revendication 6, dans lequel le polymère est un polyalkylèneglycol contenant des motifs en C₂ et/ou C₃.

8. Procédé suivant la revendication 7, dans lequel le polymère est un polyéthylèneglycol.

9. Procédé suivant la revendication 5, dans lequel le polymère est une protéine.

10. Procédé suivant l'une quelconque des revendications 2 à 9, dans lequel ladite protéine conjuguée ou ledit peptide conjugué est un peptide, un polypeptide, un anticorps, un fragment d'anticorps, une enzyme, une cytokine, une chimiokine, un récepteur, un facteur sanguin, une hormone peptidique, une toxine, une protéine de transcription, ou une protéine multimère.

11. Procédé suivant la revendication 10, dans lequel ladite protéine conjuguée ou ledit peptide conjugué est un anticorps ou un fragment d'anticorps.

12. Procédé suivant l'une quelconque des revendications 2 à 11, dans lequel chaque Q représente indépendamment une liaison directe, un groupe alkylène en C₁ à C₁₀, ou un groupe aryle ou hétéroaryle facultativement substitué, n'importe lequel de ces groupes pouvant être terminé ou interrompu par un ou plusieurs atomes d'oxygène, atomes de soufre, groupes -NR dans lesquels R représente un atome d'hydrogène ou un groupe alkyle ou aryle, groupes céto, groupes -O-CO- et/ou groupes -CO-O-.

13. Procédé suivant la revendication 12, dans lequel Q représente un groupe alkylène ou une liaison directe.

14. Procédé suivant l'une quelconque des revendications 2 à 13, dans lequel W représente un groupe céto ou aldéhyde CO, un groupe ester -O-CO- ou un groupe sulfone -SO₂-, ou un groupe obtenu par réduction d'un tel groupe ; ou dans lequel X-Q-W-, conjointement, représentent un groupe électrophile.

15. Procédé suivant l'une quelconque des revendications 2 à 14, dans lequel X représente un polymère et X'-Q-représente H-.

16. Composé de formule générale 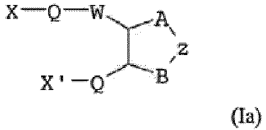
dans laquelle un de X et X' représente un polymère et l'autre représente un atome d'hydrogène ;
chaque Q représente indépendamment un groupe de liaison ;
W représente un groupement électrophile ou un groupement pouvant être préparé par réduction d'un groupement électrophile ; ou, si X' représente un polymère, X-Q-W-, conjointement, peuvent représenter un groupe électrophile ; et, en outre, si X représente un polymère, X' et le groupe électrophile W, conjointement avec les atomes intermédiaires, peuvent former un noyau ;
Z représente une protéine ou un peptide lié à A et B par l'intermédiaire de résidus histidine présents dans un marqueur polyhistidine fixé à ladite protéine ou audit peptide et contenant 2 à 12 résidus histidine ;
A représente une chaîne alkylène ou alcénylène en C₁ à C₅ ; et
B représente une liaison ou une chaîne alkylène ou alcénylène en C₁ à C₄ ;
ledit composé ayant été préparé par un procédé suivant l'une quelconque des revendications 1 à 15.

17. Composition pharmaceutique comprenant un composé suivant la revendication 16, conjointement avec un support pharmaceutiquement acceptable.

18. Composé suivant la revendication 16, ou composition pharmaceutique suivant la revendication 17, pour une utilisation en thérapie.
